(19) Europäisches Patentamt
European Patent Office
Office européen des brevets

(11) EP 2 912 149 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**25.12.2024 Patentblatt 2024/52**

(45) Hinweis auf die Patenterteilung:
**14.06.2017 Patentblatt 2017/24**

(21) Anmeldenummer: **13780142.9**

(22) Anmeldetag: **23.10.2013**

(51) Internationale Patentklassifikation (IPC):
***C10L 1/222*** *(2006.01)* ***C07C 209/20*** *(2006.01)*
***C07C 211/63*** *(2006.01)*

(52) Gemeinsame Patentklassifikation (CPC):
**C10L 1/2225; C07C 215/40; C07D 207/04; C07D 209/12; C10L 1/143; C10L 1/232; C10L 1/2383; C10L 10/04; C10L 10/06; C10M 133/08; C10M 133/44; C10M 133/54; C10M 133/58; C10M 141/06; C10M 161/00;**
(Forts.)

(86) Internationale Anmeldenummer:
**PCT/EP2013/072169**

(87) Internationale Veröffentlichungsnummer:
**WO 2014/064151 (01.05.2014 Gazette 2014/18)**

(54) **VERWENDUNG VON QUATERNISIERTEN AMMONIUMSALZE VON HYDROCARBYLEPOXIDEN ALS ADDITIVE IN KRAFT- UND SCHMIERSTOFFEN**

USE OF QUATERNISED AMMONIUM SALTS OF HYDROCARBYL EPOXIDES AS ADDITIVES IN FUELS AND LUBRICANTS

UTILISATION DES SELS D’AMMONIUM QUATERNISÉS D’HYDROCARBYLÉPOXYDES COMME ADDITIF DE CARBURANT ET LUBRIFIANTS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.10.2012 EP 12189538**

(43) Veröffentlichungstag der Anmeldung:
**02.09.2015 Patentblatt 2015/36**

(73) Patentinhaber: **BASF SE**
**67056 Ludwigshafen am Rhein (DE)**

(72) Erfinder:
- **HANSCH, Markus**
  **67346 Speyer (DE)**
- **BÖHNKE, Harald**
  **68167 Mannheim (DE)**
- **GRABARSE, Wolfgang**
  **68163 Mannheim (DE)**
- **KÖNIG, Hannah Maria**
  **76297 Stutensee (DE)**
- **LANGE, Arno**
  **67098 Bad Dürkheim (DE)**
- **VÖLKEL, Ludwig**
  **67117 Limburgerhof (DE)**

(74) Vertreter: **Meissner Bolte Partnerschaft mbB**
**Patentanwälte Rechtsanwälte**
**Postfach 86 06 24**
**81633 München (DE)**

(56) Entgegenhaltungen:
**EP-A1- 2 604 674**
**EP-A1- 2 604 674**
**WO-A1-2010/096168**
**WO-A1-2010/101801**
**WO-A1-2011/110860**
**WO-A1-2013/117616**
**WO-A1-2015/011506**
**WO-A1-2015/011507**
**WO-A2-2006/135881**
**WO-A2-2006/135881**
**WO-A2-2008/027881**
**DE-A1- 102005 041 789**
**DE-A1- 102005 041 789**
**US-A- 4 621 141**
**US-A- 4 621 141**
**US-A1- 2008 060 608**
**US-A1- 2012 010 112**
**US-A1- 2013 118 062**

(52) Gemeinsame Patentklassifikation (CPC): (Forts.)
C10L 1/1881; C10L 1/1888; C10L 1/189;
C10L 2270/023; C10L 2270/026; C10M 2207/121;
C10M 2207/125; C10M 2207/144; C10M 2207/18;
C10M 2215/042; C10M 2215/223; C10M 2215/26;
C10M 2215/30; C10N 2030/04; C10N 2040/253;
C10N 2040/255

## Beschreibung

Die vorliegende Erfindung betrifft die Verwendung quaternisierter Ammoniumsalze von Hydrocarbylepoxiden als Kraftstoffadditiv, wie insbesondere als Detergenzadditiv; zur Verringerung oder Verhinderung von Ablagerungen in den Einspritzsystemen von direkteinspritzenden Dieselmotoren, insbesondere in Common-Rail-Einspritzsystemen, zur Verringerung des Kraftstoffverbrauches von direkteinspritzenden Dieselmotoren, insbesondere von Dieselmotoren mit Common-Rail-Einspritzsystemen, und zur Minimierung des Leistungsverlustes (power loss) in direkteinspritzenden Dieselmotoren, insbesondere in Dieselmotoren mit Common-Rail-Einspritzsystemen; sowie als Additiv für Ottokraftstoffe, insbesondere zum Betrieb von DISI Motoren.

## Stand der Technik:

Bei direkteinspritzenden Dieselmotoren wird der Kraftstoff durch eine direkt in den Brennraum des Motors reichende Mehrloch-Einspritzdüse eingespritzt und feinst verteilt (vernebelt), anstatt wie beim klassischen (Kammer-) Dieselmotor in eine Vor- oder Wirbelkammer eingeführt zu werden. Der Vorteil der direkteinspritzenden Dieselmotoren liegt in ihrer für Dieselmotoren hohen Leistung und einem dennoch geringen Verbrauch. Außerdem erreichen diese Motoren ein sehr hohes Drehmoment schon bei niedrigen Drehzahlen.

Zurzeit werden im Wesentlichen drei Verfahren eingesetzt, um den Kraftstoff direkt in den Brennraum des Dieselmotors einzuspritzen: die konventionelle Verteilereinspritzpumpe, das Pumpe-Düse-System (Unit-Injector-System bzw. Unit-Pump-System) und das Common-Rail-System.

Beim Common-Rail-System wird der Dieselkraftstoff von einer Pumpe mit Drücken bis zu 2000 bar in eine Hochdruckleitung, die Common-Rail gefördert. Ausgehend von der Common-Rail laufen Stichleitungen zu den verschiedenen Injektoren, die den Kraftstoff direkt in den Brennraum injizieren. Dabei liegt auf der Common-Rail stets der volle Druck an, was eine Mehrfacheinspritzung oder eine spezielle Einspritzform ermöglicht. Bei den anderen Injektionssystemen ist dagegen nur eine geringere Variation der Einspritzung möglich. Die Einspritzung beim Common-Rail wird im Wesentlichen in drei Gruppen unterteilt: (1.) Voreinspritzung, durch die im Wesentlichen eine weichere Verbrennung erreicht wird, so dass harte Verbrennungsgeräusche ("Nageln") vermindert werden und der Motorlauf ruhig erscheint; (2.) Haupteinspritzung, die insbesondere für einen guten Drehmomentverlauf verantwortlich ist; und (3.) Nacheinspritzung, die insbesondere für einen geringen $NO_x$-Wert sorgt. Bei dieser Nacheinspritzung wird der Kraftstoff in der Regel nicht verbrannt, sondern durch Restwärme im Zylinder verdampft. Das dabei gebildete Abgas-/Kraftstoffgemisch wird zur Abgasanlage transportiert, wo der Kraftstoff in Gegenwart geeigneter Katalysatoren als Reduktionsmittel für die Stickoxide $NO_x$ wirkt.

Durch die variable, zylinderindividuelle Einspritzung kann beim Common-Rail-Einspritzsystem der Schadstoffausstoß des Motors, z.B. der Ausstoß von Stickoxiden ($NO_x$), Kohlenmonoxid (CO) und insbesondere von Partikeln (Ruß), positiv beeinflusst werden. Dies ermöglicht beispielsweise, dass mit Common-Rail-Einspritzsystemen ausgerüstete Motoren der Euro 4-Norm theoretisch auch ohne zusätzlichen Partikelfilter genügen können.

In modernen Common-Rail-Dieselmotoren können sich unter bestimmten Bedingungen, beispielsweise bei Verwendung von biodieselhaltigen Kraftstoffen oder von Kraftstoffen mit Metall-Verunreinigungen wie Zink-Verbindungen, Kupfer-Verbindungen, Bleiverbindungen und weiteren Metallverbindungen, an den Injektoröffnungen Ablagerungen bilden, die das Einspritzverhalten des Kraftstoffs negativ beeinflussen und dadurch die Performance des Motors beeinträchtigen, d.h. insbesondere die Leistung verringern, aber zum Teil auch die Verbrennung verschlechtern. Die Bildung von Ablagerungen wird durch bauliche Weiterentwicklungen der Injektoren, insbesondere durch die Veränderung der Geometrie der Düsen (engere, konische Öffnungen mit abgerundetem Auslass) noch verstärkt. Für eine dauerhaft optimale Funktionsweise von Motor und Injektoren müssen solche Ablagerungen in den Düsenöffnungen durch geeignete Kraftstoffadditive verhindert oder reduziert werden

In den Einspritzsystemen moderner Dieselmotoren verursachen Ablagerungen signifikante Performance-Probleme. Weit verbreitet ist die Erkenntnis, dass derartige Ablagerungen in den Sprühkanälen zu einer Verringerung des Kraftstoffflusses und damit zu Leistungsverlusten (power loss) führen können. Ablagerungen an der Injektorspitze beeinträchtigen dagegen die optimale Ausbildung von Kraftstoff-Sprühnebel und bedingen dadurch eine verschlechterte Verbrennung und damit verbunden höhere Emissionen und vermehrten Kraftstoffverbrauch. Im Gegensatz zu diesen herkömmlichen, "äußeren" Ablagerungsphänomenen bereiten auch "interne" Ablagerungen (zusammengefasst als innere Diesel-Injektor-Ablagerungen (IDID)) in bestimmten Teilen der Injektoren, wie an der Düsennadel, am Steuerkolben, am Ventilkolben, am Ventilsitz, an der Ansteuereinheit und an den Führungen dieser Komponenten zunehmend Performance-Probleme. Herkömmliche Additive zeigen eine unzureichende Wirkung gegen diese IDIDs.

Aus der US 4,248,719 sind quaternisierte Ammoniumsalze beschrieben, welche durch Umsetzung eines Alkenylsuccinimids mit einem Monocarbonsäureester hergestellt werden und als Dispergiermittel in Schmierölen zur Verhinderung von Schlammbildung Anwendung finden. Insbesondere ist beispielsweise die Umsetzung von Polyisobutylbernsteinsäureanhydrid (PIBSA) mit N,N-Dimethylaminopropylamin (DMAPA) und Quaternisierung mit Methylsalicylat beschrieben. Eine Anwendung in Kraftstoffen, insbesondere Dieselkraftstoffen, wird darin jedoch nicht vorgeschlagen.

Aus der US 4,171,959 sind quaternisierte Ammoniumsalze von hydrocarbylsubstituierten Succinimiden beschrieben, welche als Detergenzadditive für Ottokraftstoffzusammensetzungen geeignet sind. Zur Quaternisierung werden bevorzugt Alkylhalogenide eingesetzt. Erwähnt sind außerdem organische $C_2$-$C_8$-Hydrocarbyl-Carboxylate und - Sulfonate. Folglich weisen die gemäß dortiger Lehre bereitgestellten quaternisierten Ammoniumsalze als Gegenion entweder ein Halogenid oder ein $C_2$-$C_8$-Hydrocarbyl-Carboxylat oder ein $C_2$-$C_8$-Hydrocarbyl-Sulfonat-Gruppe auf.

Aus der EP-A-2 033 945 sind Kaltfließverbesserer bekannt, welche durch Quaternisierung spezieller tertiärer Monoamine, die wenigstens einen $C_8$-$C_{40}$-Alkyilrest tragen, mit einem $C_1$-$C_4$-Alkylester spezieller Carbonsäuren hergestellt werden. Beispiele solcher Carbonsäureester sind Dimethyloxalat, Dimethylmaleat, Dimethylphthalat und Dimethylfumarat. Andere Anwendungen als zur Verbesserung des CFPP Werts von Mitteldestillaten sind in der EP-A-2 033 945 nicht belegt.

Die WO 2006/135881 beschreibt quaternisierte Ammoniumsalze, hergestellt durch Kondensation eines Hydrocarbyl-substituierten Acylierungsmittels und einer Sauerstoff-oder Stickstoffatom-haltigen Verbindung mit tertiärer Aminogruppe, und anschließender Quaternisierung mittels Hydrocarbylepoxid in Kombination mit stöchiometrischen Mengen einer Säure, wie insbesondere Essigsäure. Weitere in der WO 2006/135881 beanspruchte Quaternisierungsmittel sind Dialkylsulfate, Benzylhalogenide und hydrocarbylsubstituierte Carbonate, wobei Dimethylsulfat, Benzylchlorid und Dimethylcarbonat experimentell untersucht wurden.

Aus der WO 2008/060888 sind quaternäre Ammoniumsalze von polyalkensubstituierten Aminen bekannt, die als Detergensadditive in Kraftstoffzusammenstezungen zur Verringerung von Einlasssystemablagerungen verwendet werden. Bevorzugte Verbindungen werden durch Hydroformylierung von Polyisobuten oder Chlorierung von Polyisobuten und anschließende Umsetzung mit einem Diamin, gefolgt von der Quaternisierung des so erzeugten Polyisobutendiamins mittels herkömmlicher Quaternisierungsmittel, wie Dimethylsulfat, Benzylchlorid, oder Styrolepoxid/Säure hergestellt.

Die bisher bekannten quaternisierten Verbindungen sind mit relativ hohem Syntheseaufwand herzustellen. Es bestand daher die Aufgabe, quaternisierte Kraftstoffadditive bereitzustellen, die einerseits einfacher herzustellen sind und andererseits zufriedenstellende Additiveigenschaften besitzen

## Kurze Beschreibung der Erfindung:

Es wurde nun überraschenderweise gefunden, dass obige Aufgabe durch die in den Patentansprüchen definierte Verwendung quaternisierter Ammoniumsalze von Hydrocarbylepoxiden in Kraftstoffzusammensetzungen gelöst wird.

Überraschenderweise sind die erfindungsgemäßen Additive wie insbesondere durch die beiliegenden Anwendungsbeispiele veranschaulicht, nicht nur, ausgehend von den korrespondierenden Hydrocarbyl-Epoxid-Vorstufen in einfacher Weise herzustellen, sondern zeigen auch überraschenderweise zufriedenstellende Additiveigenschaften, wie insbesondere beim Betrieb in modernen Dieselmotoren.

## Figurenbeschreibung:

Figur 1 zeigt den Ablauf des einstündiges Motorentestzyklus gemäß CEC F-098-08.

## Detaillierte Beschreibung der Erfindung:

### A1) Spezielle Ausführungsformen

Die vorliegende Erfindung ist anhand der erteilten Ansprüchen definiert und betrifft insbesondere folgende Ausführungsformen:

1. Verwendung wenigstens eines eine quaternisierte Stickstoffverbindung umfassenden Reaktionsprodukts oder eine aus dem Reaktionsprodukt durch Aufreinigung erhaltene, eine quaternisierte Stickstoffverbindung enthaltende wie in Anspruch 1 definierte Teilfraktion davon, wobei das Reaktionsprodukt erhältlich ist durch Umsetzung wenigstens eines Hydrocarbylepoxids der allgemeinen Formel I

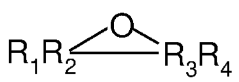

(I)

worin

wenigstens einer der Reste $R_1$ und $R_2$ für einen geradkettigen oder verzweigten, gesättigten oder ungesättigten wie in Anspruch 1 definierten langkettigen Hydrocarbylrest, z.B. Polyalkylen-Rest steht und der andere der beiden Reste gegebenenfalls für H oder einen wie in Anspruch 1 definierten kurzkettigen Hydrocarbylrest (insbesondere $C_1$-$C_4$ Alkyl) steht; und
die Reste $R_3$ und $R_4$ gleich oder verschieden sind und für H oder einen kurzkettigen Hydrocarbylrest (insbesondere $C_1$-$C_4$ Alkyl) stehen;
mit wenigstens einem tertiären Amin der allgemeinen Formel II

$$R_aR_bR_cN \qquad (II)$$

worin
$R_a$, $R_b$ und $R_c$ unabhängig voneinander für einen geradkettigen oder verzweigten gesättigten oder ungesättigten, gegebenenfalls substituierten Hydrocarbylrest, insbesondere kurzkettigen Hydrocarbylrest, oder für Alkyl oder Alkenyl, oder zwei der Reste $R_a$, $R_b$ und $R_c$, zusammen mit dem Stickstoffatom, an das sie gebunden sind, einen gegebenenfalls substituierten heterocyclischen Ring, insbesondere 5- bis 7-gliedrigen, gesättigten oder ungesättigten nicht-aromatischen oder aromatischen heterocyclischen Ring, der gegebenenfalls wenigstens ein weiteres Ringheteroatom, wie O,S oder N, tragen kann, bilden; wobei der Hydrocarbylrest im Amin der Formel (II) ein kurzkettiger Hydrocarbylrest ist und für geradkettiges oder verzweigtes $C_1$-$C_7$-Alkyl oder $C_2$-$C_7$-Alkenyl, gegebenenfalls unterbrochen durch eine oder mehrere Heteroatomgruppen oder mehrfach substituiert steht;
sowie in Gegenwart wenigstens einer Säure der Formel III

$$H^+A^- \qquad (III)$$

worin
$A^-$ für das Anion wenigstens einer ein- oder mehrwertigen, anorganischen oder organischen, natürlichen oder synthetischen Säure steht;
wobei die optionalen Substituenten der Reste ausgewählt sind unter -OH;
als Kraftstoffadditiv, wie in den Ansprüchen definiert.
Die quaternisierte Stickstoffverbindung kann sowohl von einem einzelnen Epoxid der Formel I oder einem Gemisch mehrerer, voneinander verschiedenen Epoxide der Formel I abgeleitet sein.

2. Verwendung nach Ausführungsform 1, wobei das Reaktionsprodukt wenigstens eine Verbindung der allgemeinen Formel IV

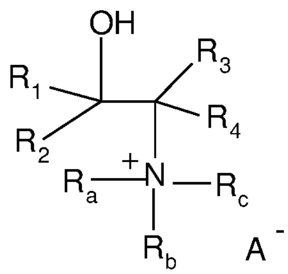

(IV)

umfasst,
worin
die Reste $R_1$, $R_2$, $R_3$, $R_4$, $R_a$, $R_b$, $R_c$ und A die oben angegebenen Bedeutungen besitzen.

3. Verwendung nach einer der Ausführungsformen 1 und 2, wobei das Amin der allgemeinen Formel II ausgewählt ist unter Verbindungen der allgemeinen Formel II worin einer der Reste $R_a$, $R_b$ und $R_c$ für einen $C_1$-$C_4$-Alkylrest steht und die beiden anderen Reste zusammen mit dem Stickstoffatom an das sie gebunden sind einen 5- oder 6-gliedrigen heterocyclischen, gesättigten oder ungesättigten Ring, der gegebenenfalls wenigstens ein weiteres Ringheteroatom, wie O, S oder N, tragen kann, bilden.

4. Verwendung nach einer der vorhergehenden Ausführungsformen, wobei die Verbindung der allgemeine Formel I ein Polyalkylenpoxid ist, das man durch Epoxidierung eines Polyalkylens, insbesondere Poly-($C_2$-$C_6$)-Alkens erhält,

das ein zahlengemitteltes Molekulargewicht ($M_n$) von 800 bis 1.500 aufweist.

5. Verwendung nach einer der Ausführungsformen 1 bis 3, wobei in den Verbindungen der allgemeinen Formel I der langkettige Hydrocarbylrest für einen geradkettigen oder verzweigten, aliphatischen Kohlenwasserstoffrest mit 8 bis 40, insbesondere 10 bis 20, 10 bis 16 oder 10 bis 14 zusammenhängenden C-Atomen steht. Beispielsweise sind zu nennen $C_{12}$-$C_{16}$-Alkylepoxide. Insbesondere geeignet sind solche Verbindungen, die terminale Epoxidgruppen tragen, d.h. zwei benachbarte C-Atome am Kettenende (omega-Position) oder in omega -1 oder omega -2-Position tragen die Epoxidgruppe .

6. Verwendung nach Ausführungsform 4, wobei das Polyalkylen ein Polyisobuten mit einem einen Anteil an Vinyliden-Doppelbindungen von größer 70 Mol-%, insbesondere größer 80 Mol-% oder größer 85 Mol-% ist.

7. Verwendung nach einer der vorhergehenden Ausführungsformen, ausgewählt unter Dieselkraftstoffen, Biodieselkraftstoffen, Ottokraftstoffen, und Alkanolhaltigen Ottokraftstoffen.
Hierin beschrieben werden außerdem:
quaternisierte Stickstoffverbindung umfassend ein Reaktionsprodukt gemäß der Definition in einer der Ausführungsformen 1 bis 5, insbesondere eine Verbindung der Formel IV.

8. Quaternisierte Stickstoffverbindung der obigen allgemeinen Formel IV.

9. Verfahren zur Herstellung einer quaternisierter Stickstoffverbindung nach Ausführungsform 7 oder 8,

umfassend die Umsetzung wenigstens eines Hydrocarbylepoxids der allgemeinen Formel I

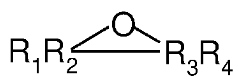 (I)

worin
wenigstens einer der Reste $R_1$ und $R_2$ für einen geradkettigen oder verzweigten, gesättigten oder ungesättigten langkettigen Hydrocarbylrest, z.B. Polyalkylen-Rest steht und der andere der beiden Reste gegebenenfalls für H oder einen kurzkettigen Hydrocarbylrest (insbesondere $C_1$-$C_4$ Alkyl) steht; und
die Reste $R_3$ und $R_4$ gleich oder verschieden sind und für H oder einen kurzkettigen Hydrocarbylrest (insbesondere $C_1$-$C_4$ Alkyl) stehen;
mit wenigstens einem tertiären Amin der allgemeinen Formel II

$$R_aR_bR_cN \qquad (II)$$

worin
$R_a$, $R_b$ und $R_c$ unabhängig voneinander für einen geradkettigen oder verzweigten gesättigten oder ungesättigten, gegebenenfalls substituierten Hydrocarbylrest, insbesondere kurzkettigen Hydrocarbylrest, oder für Alkyl oder Alkenyl, insbesondere $C_1$-$C_{24}$-Alkyl oder $C_2$-$C_{24}$-Alkenyl, stehen, oder zwei der Reste $R_a$, $R_b$ und $R_c$, zusammen mit dem Stickstoffatom, an das sie gebunden sind, einen gegebenenfalls substituierten heterocyclischen Ring, insbesondere 5- bis 7-gliedrigen, gesättigten oder ungesättigten nicht-aromatischen oder aromatischen heterocyclischen Ring, der gegebenenfalls wenigstens ein weiteres Ringheteroatom, wie O, S oder N, tragen kann, bilden;
sowie in Gegenwart einer Säure der Formel III

$$H^+A^- \qquad (III)$$

worin
$A^-$ für das Anion wenigstens einer ein- oder mehrwertigen, anorganischen oder organischen, natürlichen oder synthetischen Säure steht..

10. Verfahren nach Ausführungsform 9, wobei das Reaktionsprodukt wenigstens eine Verbindung der allgemeinen Formel IV

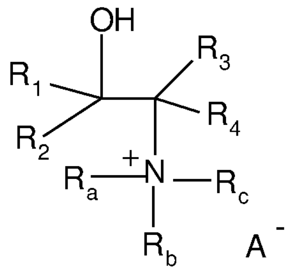

(IV)

umfasst, worin die Reste $R_1$, $R_2$, $R_3$, $R_4$, $R_a$, $R_b$, $R_c$ und A die oben angegebenen Bedeutungen besitzen.

11. Verfahren nach einer der Ausführungsformen 9 und 10, wobei das Amin der allgemeinen Formel II ausgewählt ist unter Tri-$C_1$-$C_{24}$- oder Tri-$C_4$-$C_{12}$-Alkylaminen oder Verbindungen der allgemeinen Formel II worin einer der Reste $R_a$, $R_b$ und $R_c$ für einen $C_1$-$C_4$-Alkylrest steht und die beiden anderen Reste zusammen mit dem Stickstoffatom an das sie gebunden sind einen 5- oder 6-gliedrigen heterocyclischen, gesättigten oder ungesättigten Ring, der gegebenenfalls wenigstens ein weiteres Ringheteroatom, wie O,S oder N, tragen kann, bilden.

12. Verfahren nach einer der vorhergehenden Ausführungsformen, wobei die Verbindung der allgemeine Formel I ein Polyalkylenpoxid ist, das man durch Epoxidierung eines Polyalkens, insbesondere Poly-($C_2$-$C_6$)-alkens, erhält, das ein zahlengemitteltes Molekulargewicht ($M_n$) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500 aufweist.

13. Verfahren nach Ausführungsform 12, wobei das Polyalkylen ein Polyisobuten mit einem einen Anteil an Vinyliden-Doppelbindungen von größer 70 Mol-%, insbesondere größer 80 Mol-% oder größer 85 Mol-% ist.
Die Erfindung betrifft weiterhin:

14. Verwendung einer quaternisierten Stickstoffverbindung nach Anspruch 7 oder 8 oder hergestellt nach einer der Ausführungsformen 9 bis 13 als Kraftstoffadditiv.

15. Verwendung nach Ausführungsform 14 als Additiv zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen, bestimmt wie im Experimentellen Teil allgemein beschrieben (Powerloss-KC oder Powerloss - DU oder Powerloss-DU,CU).

16. Verwendung nach Ausführungsform 15 als Ottokraftstoffadditiv zur Verringerung von Ablagerungen im Einlasssystem eines Ottomotors, wie insbesondere DISI und PFI (Port Fuel injector) -Motoren.

17. Verwendung nach Ausführungsform 16 als Dieselkraftstoffadditiv zur Verringerung und/oder Vermeidung von Ablagerungen in den Einspritzsystemen, wie insbesondere der Internal Diesel Injector Deposits (IDID) und / oder von Ventilkleben in direkteinspritzenden Dieselmotoren, insbesondere in Common-Rail-Einspritzsystemen, jeweils bestimmt wie im Experimentellen Teil allgemein beschrieben (XUD-9 oder IDIDI).
Weiterhin sind hierin beschrieben:

18. Additivkonzentrat, enthaltend in Kombination mit weiteren Diesel- oder Ottokraftstoffadditiven oder Schmierstoffadditiven wenigstens eine quaternisierte Stickstoffverbindung gemäß der Definition in Ausführungsform 7 oder 8 oder hergestellt nach einer der Ausführungsformen 9 bis 13.

**A2) Allgemeine Definitionen**

Werden keine gegenteiligen Angaben gemacht, so gelten folgende allgemeine Bedeutungen:
"Hydrocarbyl" ist breit auszulegen und umfasst sowohl langkettige als auch kurzkettige, cyclische oder nichtcyclische gerade oder verzweigte, gesättigte oder ungesättigte, aliphatische, cycloaliphatische, oder aromatische (z.B. Aryl), insbesondere aliphatische, Kohlenwasserstoffreste mit 1 bis 50 Kohlenstoffatomen, welche ggf. zusätzlich Heteroatome, wie z.B. O, N, NH,S, in ihrer Kette enthalten können.

"Langkettige" Hydrocarbylreste stehen für geradkettige oder verzweigte Kohlenwasserstoffreste und weisen 7

bis 50 oder 8 bis 40 oder 10 bis 20 Kohlenstoffatomen, welche ggf. zusätzlich Heteroatome, wie z.B. O, N, NH,S, in ihrer Kette enthalten können. In einer besonderen Ausführungsform sind keine Heteroatome enthalten. Weiterhin können die Reste ein- oder mehrfach ungesättigt sein und ein oder mehrere nichtkumulierte, wie z.B. 1 bis 5, wie 1, 2 oder 3 C-C Doppelbindungen oder C-C- Dreifachbindungen, insbesondere 1, 2 oder 3 Doppelbindungen, aufweisen. Sie können natürlichen oder synthetischen Ursprungs sein. Sie können auch ein zahlengemitteltes Molekulargewicht ($M_n$) von 85 bis 20.000, wie z.B. 113 bis 10.000, oder 200 bis 10.000 oder 350 bis 5.000, wie z.B. 350 bis 3.000, 500 bis 2.500, 700 bis 2.500, oder 800 bis 1.500 aufweisen Sie sind dann insbesondere im Wesentlichen aus $C_{2-6}$-, insbesondere $C_{2-4}$-Monomerbausteinen, wie Ethylen, Propylen, n- oder iso-Butylen oder Mischungen davon aufgebaut, wobei die verschiedenen Monomere statistisch verteilt oder als Blöcke einpolymerisiert enthalten sein können. Derartige langkettige Hydrocarbylreste werden auch als Polyalkylenreste oder Poly-$C_{2-6}$- oder Poly-$C_{2-4}$-alkylenreste bezeichnet. Geeignete langkettige Hydrocarbylreste und deren Herstellung sind beispielsweise auch beschreiben in der WO2006/135881 und der dort zitierten Literatur.

Beispiele für besonders brauchbare Polyalkylen-Reste sind Polyisobutenyl-Reste, abgeleitet von sogenannten "hochreaktiven" Polyisobutenen, die sich durch einen hohen Gehalt an terminal angeordneten Doppelbindungen auszeichnen. Terminal angeordnete Doppelbindungen sind dabei alpha-olefinische Doppelbindungen der Formel V

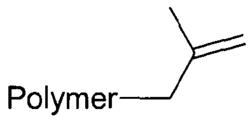 (V)

welche zusammen auch als Vinyliden-Doppelbindungen bezeichnet werden. Geeignete hochreaktive Polyisobutene sind beispielsweise Polyisobutene, die einen Anteil an Vinyliden-Doppelbindungen von größer 70 Mol-%, insbesondere größer 80 Mol-% oder größer 85 Mol-% aufweisen. Bevorzugt sind insbesondere Polyisobutene, die einheitliche Polymergerüste aufweisen. Einheitliche Polymergerüste weisen insbesondere solche Polyisobutene auf, die zu wenigstens 85 Gew.-%, vorzugsweise zu wenigstens 90 Gew.-% und besonders bevorzugt zu wenigstens 95 Gew.-% aus Isobuteneinheiten aufgebaut sind. Vorzugsweise weisen solche hochreaktiven Polyisobutene ein zahlenmittleres Molekulargewicht in dem oben genannten Bereich auf. Darüber hinaus können die hochreaktiven Polyisobutene eine Polydispersität im Bereich von 1,05 bis 7, insbesondere von etwa 1,1 bis 2,5, wie z.B. von kleiner 1,9 oder kleiner 1,5, aufweisen. Unter Polydispersität versteht man den Quotienten aus gewichtsmittlerem Molekulargewicht Mw geteilt durch das zahlenmittlere Molekulargewicht Mn.

Besonders geeignete hochreaktive Polyisobutene sind z.B. die Glissopal-Marken der BASF SE, insbesondere Glissopal 1000 (Mn = 1000), Glissopal V 33 (Mn = 550) und Glissopal 2300 (Mn = 2300) und deren Mischungen. Andere zahlenmittlere Molekulargewichte können nach im Prinzip bekannter Weise durch Mischen von Polyisobutenen unterschiedlicher zahlenmittlerer Molekulargewichte oder durch extraktive Anreicherung von Polyisobutenen bestimmter Molekulargewichtsbereiche eingestellt werden.

"Kurzkettiges Hydrocarbyl" oder "niedermolekulares Hydrocarbyl" steht insbesondere für geradkettiges oder verzweigtes $C_1$-$C_7$-Alkyl oder $C_2$-$C_7$-Alkenyl, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

"Hydrocarbylen" steht für geradkettige oder ein- oder mehrfach verzweigte Brückengruppen mit 1 bis 10 Kohlenstoffatomen, gegebenenfalls unterbrochen durch eine oder mehrere, wie z.B. 2, 3 oder 4 Heteroatomgruppen, wie -O- oder - NH-. oder gegebenenfalls ein- oder mehrfach, wie z.B. 2, 3 oder 4-fach substituiert.

" Alkyl" oder "Niedrigalkyl" steht insbesondere für gesättigte, geradkettige oder verzweigte Kohlenwasserstoffreste mit 1 bis 4, 1 bis 5, 1 bis 6, 1 bis 7, 1 bis 10, 1 bis 16 oder 1 bis 24 Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, n-Octyl, n-Nonyl, n-Decyl, n-Undecyl, n-Tridecyl, n-Tetradecyl, n-Pentadecyl und n-Hexadecyl, Octadecyl, Docosanyl sowie die ein- oder mehrfach verzweigten Analoga davon. Niedrigalkyl steht insbesondere für Reste mit 1 bis 4, 1 bis 5, 1 bis 6, oder 1 bis 7 C-Atomen.

" Hydroxyalkyl" steht insbesondere für die ein- oder mehrfach, insbesondere einfach hydroxysubstituierten Analoga obiger Alkyl- oder Niedrigalkylgruppen.

" Alkenyl" steht für ein- oder mehrfach, insbesondere einfach ungesättigte, geradkettige oder verzweigte Kohlenwasserstoffreste mit 2 bis 4, 2 bis 6, 2 bis 7, 2 bis 10, 2 bis 16 oder 2 bis 24 Kohlenstoffatomen und einer Doppelbindung in einer beliebigen Position, z. B. $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl,
1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl, sowie, soweit noch nicht genannt, die einfach ungesättigten Analoga obiger Alkylreste. Niedrigalkenyl steht insbesondere für Reste mit 2 bis 4, 2 bis 5,2 bis 6, oder 2 bis 7 C-Atomen.

" Alkylen" steht für geradkettige oder ein- oder mehrfach verzweigte Kohlenwasserstoff-Brückengruppen mit 1 bis 10 oder 2 bis 6 Kohlenstoffatomen, wie z.B. $C_1$-$C_7$-oder $C_2$-$C_6$-Alkylengruppen ausgewählt unter $-CH_2-$, $-(CH_2)_2-$, $-(CH_2)_3-$, $-CH_2-CH(CH_3)-$, $-CH(CH_3)-CH_2-$, $-(CH_2)_4-$, $-(CH_2)_2-CH(CH_3)-$, $-CH_2-CH(CH_3)-CH_2-$, $(CH_2)_4-$, $-(CH_2)_5-$,$-(CH_2)_6$, $-(CH_2)_7-$, $-CH(CH_3)-CH_2-CH_2-CH(CH_3)-$ oder - $CH(CH_3)-CH_2-CH_2-CH_2-CH(CH_3)-$ oder $C_1$-$C_4$- oder $C_2$-$C_4$-Alkylengruppen ausgewählt unter $-CH_2-$, $-(CH_2)_2-$,$-(CH_2)_3-$, $-CH_2-CH(CH_3)-$, $-CH(CH_3)-CH_2-$, $-(CH_2)_4-$, $-(CH_2)_2-CH(CH_3)-$, $-CH_2-CH(CH_3)-CH_2-$.

"Alkenylen" steht für die ein- oder mehrfach, insbesondere einfach ungesättigten Analoga obiger Alkylengruppen mit 2 bis 10 Kohlenstoffatomen, insbesondere für $C_2$-$C_7$-Alkenylene oder $C_2$-$C_4$-Alkenylen, wie -CH=CH-, $-CH=CH-CH_2-$, - $CH_2-CH=CH-$, $-CH=CH-CH_2-CH_2-$, $-CH_2-CH=CH-CH_2-$, $-CH_2-CH_2-CH=CH-$, $-CH(CH_3)-CH=CH-$, $-CH_2-C(CH_3)=CH-$.

" Cycloalkyl" steht für carbocyclische Reste mit 3 bis 20 Kohlenstoffatomen, wie z.B. $C_3$-$C_{12}$-Cycloalkyl, wie Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, sowie Cyclopropyl-methyl, Cyclopropyl-ethyl, Cyclobutyl-methyl, Cyclobutyl-ethyl, Cyclopentyl-methyl, Cyclopentyl-ethyl, Cyclohexyl-methyl oder $C_3$-$C_7$-Cycloalkyl, wie Cyclopropyl, Cyclobutyl, Cyclopentyl, Cyclohexyl, Cycloheptyl, Cyclopropyl-methyl, Cyclopropyl-ethyl, Cyclobutyl-methyl, Cyclopentylethyl, Cyclohexyl-methyl, wobei die Anbindung an den Rest des Moleküls über jegliches geeignetes C-Atom erfolgen kann.

" Cycloaliphatische " Reste umfassen insbesondere obige Cycloalkyl Reste sowie die einfach ungesättigten Analoga ("Cycloalkenyl") davon.

-" Aryl" steht für ein- oder mehrkernige, vorzugsweise ein- oder zweikernige, gegebenenfalls substituierte aromatische Reste mit 6 bis 20 wie z.B. 6 bis 10 RingKohlenstoffatomen, wie z.B. Phenyl, Biphenyl, Naphthyl wie 1- oder 2-Naphthyl, Tetrahydronaphthyl, Fluorenyl, Indenyl und Phenathrenyl. Diese Arylreste können gegebenenfalls 1, 2, 3, 4, 5 oder 6 gleiche oder verschiedene Substituenten tragen.

"Aralkyl" steht für die ein- oder mehrfach Aryl-substituierten Analoga obiger Alkyl-oder Niedrigalkylgruppen.

"Substituenten "für hierin angegebene Reste, sind insbesondere, wenn keine anderen Angaben gemacht werde, ausgewählt sind unter Ketogruppen, - COOH, -COO-Alkyl, - OH, -SH, -CN, Amino, $-NO_2$, Alkyl, oder Alkenylgruppen.

"Mn" steht für das zahlenmittlere Molekulargewicht und wird in herkömmlicher Weise bestimmt; insbesondere beziehen sich solche Angaben auf Mn-Werte, z.B. bestimmt durch Gelpermeationschromatographie;
insbesondere bestimmt durch Relativmethoden, wie der Gelpermeationschromatographie mit THF als Eluent und Polystyrol-Standards oder Absolutmethoden, wie der Dampfphasenosmometrie unter Verwendung von Toluol als Lösungsmittel.

"Mw" steht für das gewichtsmittlere Molekulargewicht und wird in herkömmlicher Weise bestimmt; insbesondere beziehen sich solche Angaben auf Mw-Werte, bestimmt durch Relativmethoden, wie der Gelpermeationschromatographie mit THF als Eluent und Polystyrol-Standards oder Absolutmethoden, wie der Lichtstreuung.

Der "Polymerisationsgrad" bezeichnet gewöhnlich den zahlenmäßigen mittleren Polymerisationsgrad (Bestimmungsmethode Gelpermeationschromatographie mit THF als Eluent und Polystyrol-Standards; oder GC-MS Kopplung).

A3) Hydrocarbylepoxide der Formel (I)

Hydrocarbylepoxide der obigen Formel I sind an sich bekannte Verbindungen (z.B. beschrieben in der WO 2007/025700 oder EP-A-1 422 246) oder sind in an sich bekannter Weise herstellbar (vgl. z.B. Sienel, G., Rieth, R. and Rowbottom, K. T. 2000. Epoxides. Ullmann's Encyclopedia of Industrial Chemistry).

Beispiele für geeignete Ausgangsstoffe zur Epoxid-Herstellung sind grundsätzlich alle Verbindungen aus der an

sich bekannten Verbindungsklasse der Polyalkene, wie sie z.B. beschrieben sind in Koch, H., Mawer, R. L. and Immel, W. 2011. Polybutenes. Ullmann's Encyclopedia of Industrial Chemistry, James L. White, Dongman Choi.2004.Polyolefins: Processing, Structure Development, and Properties, Hanser.

Insbesondere sindnichtlimitierende Beispiele sogenannte "hochreaktiven" Polyisobutene, die sich durch einen hohen Gehalt an terminal angeordneten Doppelbindungen der obigen Formel V auszeichnen. Auf obige Offenbarung hochreaktiver Polyisobutene wird hier nochmals ausdrücklich Bezug genommen.

Die Herstellung brauchbarer Epoxide wird anhand der folgenden Beschreibung der Herstellung eines Polybutenepoxids exemplarisch erläutert.

Zu einer Lösung eines Polyisobutens in einem apolaren Lösungsmittel, wie z.B. n-Heptan werden bei Raumtemperatur Ameisensäure und Methyltrioctylammoniumchlorid ((wie z.B. beschrieben in der WO 2007/025700) gegeben. Das Reaktionsgemisch wird erhitz, wie z.B. auf eine Temperatur im Bereich von 50 bis 90 wie z.B. etwa 75°C, und Wasserstoffperoxid-Lösung wird langsam zu getropft, wobei die voreingestellte Temperatur gehalten wird. Das Reaktionsgemisch wird für anschließend über einen ausreichenden Zeitraum , wie z.B. 1 bis 36 Stunden, wie etwa 10 h bei der gleichen Temperatur gerührt, auf Raumtemperatur abgekühlt und die Phasen werden getrennt. Die organische Phase wird gewaschen, wie z. B. sukzessive mit wässriger $NaHSO_3$-Lösung, gesättigter $NaHCO_3$-Lösung und Wasser gewaschen. Die organische Phase wird über $Na_2SO_4$ getrocknet und das Lösungsmittel wird entfernt.

**A4) Tertiäre Amine der Formel (II)**

Tertiäre Amine der Formel (II) sind ebenfalls an sich bekannte Verbindungen, aus der Gruppe der aliphatischen oder aromatischen Amine. Als Beispiele sind zu nennen:

a) Nichtzyklische tertiäre Amine wie:

Amine der allgemeinen Formel II worin die Reste $R_a$, $R_b$ und $R_c$ unabhängig voneinander für $C_1$-$C_{24}$-Alkyl, wie Methyl, Ethyl, n-Propyl, Isopropyl, n-Butyl, Isobutyl, sec-butyl, tert-Butyl, n-Pentyl, Isopentyl, n-Hexyl, Isohexyl, oder $C_3$- to $C_{12}$-Cycloalkyl wie Cyclopentyl, Cyclohexyl and Cycloheptyl, , Hydroxyalkyl, wie Hydroxy-$C_1$-$C_{24}$-Alkyl, wie z.B. die einfach hydroxyl-substituierten Analoge der obigen Alkyl oder Cycloalkylgruppen; oder Aralkyl, wie z.B. Aryl-substituierte $C_1$-$C_4$-Alkyl-Reste, wie z.B. Benzyl stehen.

Nichlimitierende Beispiele hierfür sind: Trimethylamin, Triethylamin, Tripropylamin, Dimethylethylamin, Methyldiethylamin, Ethyldipropylamin, Methyldipropylamin, Dimethylpropylamin, Ethyldipropylamin, Diethylpropylamin, Tri-(n-butyl)amin, Diisopropylethylamin, Tripentylamin, Trihexylamin, Tricyclohexylamin, Ethyldicyclohexylamin. N,N-Dimethylbenzylamin, N,N-Dimethycyclohexylamin, N,N-Dimethylethanolamin, N,N-Diethylethanolamin, N,N-Dimethyethylamin, N,N-Dimethylpropylamin, N,N-Dimethylisopropylamin, N-Ethyldüsopopylamin, Tris-(2-ethylhexyl)amin, N,N,N'-N'-Tetramethyl-1,6-hexandiamin, N,N,N'-N'-Tetramethyl-1,3-propandiamin, Triethanolamin, Triisopropanolamin, N,N-Dibutylethanolamin, N-Methyldiethanolamin, N,N-Dimethylisopropanolamin, N,N-Di-(2-hydroxyethyl)anilin.

b) Zykische tertiäre Amine, wie:
Isopropydimethylamin, N-Methylpyrrolidon, N-Methylimidazol, N-Methylmorpholin, N-Methylpiperidin, N-Ethylpiperidin, N-Ethylpyrrolidin, N-Propylazepin, N-Ethylmorpholin, N,N'-dimethylpiperazin:

4-Aminopyridine, worin die H Atome der Aminogruppe durch Reste $R_a$, $R_b$ oder $R_c$ gemäß obiger Definition ersetzt wurden. Beispiele sind N,N-Dimethyl-4-aminopyridine, N,N-Diethyl-4-aminopyridin, 4-Morpholinopyridine or 4-Piperazinopyridine.

N-substituierte Imidazoles, wobei der Substituent ein $C_1$-$C_{24}$- oder $C_1$-$C_8$-Alkylrest ist, wie z.B. N-Methyl-, N-Ethyl- oder N-Propylimidazol.

**A5) Herstellung erfindungsgemäß verwendetr quaternisierter Additive der Formel (IV):**

a) Quaternisierung

Die Quaternisierung wird in an sich bekannter Weise durchgeführt

Zur Durchführung der Quaternisierung versetzt man das tertiäre Amin (II) mit wenigstens einer Epoxid-Verbindung obiger Formel (I), insbesondere in den erforderlichen stöchiometrischen Mengen, um die gewünschte Quaternisierung zu erreichen.

Falls erforderlich können die Reaktanden in einem geeigneten organischen Lösungsmittel, insbesondere alkoholischen Lösungsmittel, wie z.B. Methanol oder Ethanol, gelöst sein.

Die Umsetzung erfolgt zudem in Gegenwart stöchiometrischer Mengen einer anorganischen oder insbesondere organischen Säure der obigen Formel (III).

Insbesondere verwendet man hierzu organische Carbonsäuren, insbesondere gesättigte oder ungesättigte aliphatische oder aromatische $C_1$-$C_{20}$ Carbonsäuren, wie z.B. $C_1$-$C_4$ Monocarbonsäure, Ameisensäure, Essigsäure, Propionsäure, oder aromatische Carbonsäuren, wie Salicylsäure und Benzoesäure, oder Fettsäuren. Geeignete Fettsäuren sind geradkettige oder verzweigte, ein- oder mehrfach ungesättigten, gegebenenfalls substituierten $C_6$-$C_{30}$-Monocarbonsäuren. Beispiele für gesättigte unverzweigte Fettsäuren sind Capronsäure, Önanthsäure, Caprylsäure, Pelargonsäure, Caprinsäure, Undecansäure, Laurinsäure, Tridecansäure, Myristinsäure, Pentadecansäure, Palmitinsäure, Margarinsäure, Stearinsäure, Nonadecansäure, Arachinsäure, Behensäure, Lignocerinsäure, Cerotinsäure und Melissinsäure. Beispiele für einfach ungesättigte Fettsäuren sind Palmitoleinsäure, Ölsäure und Erucasäure. Beispiele für zweifach ungesättigte Fettsäuren sind Sorbinsäure und Linolsäure. Beispiele für dreifach ungesättigte Fettsäure sind Linolensäure und Elaeostearinsäure. Beispiele für vier- und mehrfach ungesättigte Fettsäuren sind Arachidonsäure, Clupanodonsäure und Docosahexaensäure. Beispiele für substituierte Fettsäuren sind Ricinolsäure ((R)-12-Hydroxy-(Z)-9-octadecensäure). Weitere geeignete Fettsäuren sind natürlich vorkommende Fettsäuren wie Gondosäure und Nervonsäure. Sind in den Fettsäuren Doppelbindungen enthalten, so können diese sowohl in cis- als auch in trans-Form vorliegen. Die Substituenten sind vorzugsweise ausgewählt unter Hydroxy- und Niedrigalkylgruppen, wie z.B. Methyl- und Ethylgruppen. Weiterhin können Ketogruppen oder Epoxygruppen, wie z.B. in der Vernolsäure im Kohlenwasserstoffrest enthalten sein. Weitere funktionelle Gruppen sind Cyclopropan-, Cyclopropen- und Cyclopentenringe, welche durch Verbrückung von zwei benachbarten Kohlenstoffatomen im Kohlenwasserstoffrest der Fettsäure ausgebildet werden können (vgl. Malvaliasäure und Chaulmoograsäure). Besonders zu nennen sind auch Tallölfettsäuren.

Man arbeitet hierbei typischerweise bei Temperaturen im Bereich von 50 bis 200°C, wie z.B. 90 bis 160 °C oder 100 bis 140 °C. Die Reaktionsdauer kann dabei im Bereich von wenigen Minuten oder einigen Stunden, wie z.B. etwa 10 Minuten bis zu etwa 24 Stunden liegen. Der Umsetzung kann dabei bei etwa 1 bis 20 bar, wie z.B. 1 bis 10 bar Druck, insbesondere aber in einem Autoklaven bei etwa bei Normaldruck erfolgen.

Die Reaktion erfolgt zudem insbesondere unter Inertgas, wie z.B. Stickstoff.

b) Aufarbeitung des Reaktionsgemisches

Das so gebildete Reaktionsendprodukt kann theoretisch weiter aufgereinigt oder das Lösungsmittel kann entfernt werden. Gegebenenfalls können überschüssiges Reagenz oder flüchtige Bestandteile des Reaktionsgemischs entfernt werden. Eine weitere Aufreinigung, z.B. durch Abtrennung von nicht-quaternisierten Bestandteilen, ist nicht zwingend notwendig, so dass das Reaktionsprodukt ggf nur nach Entfernung flüchtiger Bestandteile, als Additiv, gegebenenfalls nach Abmischung mit weiteren Additivkomponenten (s. unten) einsetzbar ist.

**B) Weitere Additivkomponenten**

Der mit dem erfindungsgemäßen quaternisierten Additiv additivierte Kraftstoff ist ein Ottokraftstoff oder insbesondere ein Mitteldestillat-Kraftstoff, vor allem ein Dieselkraftstoff.

Der Kraftstoff kann weitere übliche Additive zur Wirksamkeitsverbesserung und/oder Verschleißunterdrückung enthalten.

Im Falle von Dieselkraftstoffen sind dies in erster Linie übliche Detergenz-Additive, Trägeröle, Kaltfließverbesserer, Schmierfähigkeitsverbesserer (Lubricity Improver), Korrosionsinhibitoren, Demulgatoren, Dehazer, Antischaummittel, Cetanzahlverbesserer, Verbrennungsverbesserer, Antioxidantien oder Stabilisatoren, Antistatika, Metallocene, Metalldeaktivatoren, Farbstoffe und/oder Lösungsmittel.

Im Falle von Ottokraftstoffen sind dies vor allem Schmierfähigkeitsverbesserer (Friction Modifier), Korrosionsinhibitoren, Demulgatoren, Dehazer, Antischaummittel, Verbrennungsverbesserer, Antioxidantien oder Stabilisatoren, Antistatika, Metallocene, Metalldeaktivatoren, Farbstoffe und/oder Lösungsmittel.

Typische Beispiele geeigneter Co-Additive sind im folgenden Abschnitt aufgeführt:

B1) Detergenz-Additive

Vorzugsweise handelt es sich bei den üblichen Detergenz-Additiven um amphiphile Substanzen, die mindestens einen hydrophoben Kohlenwasserstoffrest mit einem zahlengemittelten Molekulargewicht ($M_n$) von 85 bis 20.000 und mindestens eine polare Gruppierung besitzen, die ausgewählt ist unter:

(Da) Mono- oder Polyaminogruppen mit bis zu 6 Stickstoffatomen, wobei mindestens ein Stickstoffatom basische Eigenschaften hat;

(Db) Nitrogruppen, gegebenenfalls in Kombination mit Hydroxylgruppen;

(Dc) Hydroxylgruppen in Kombination mit Mono- oder Polyaminogruppen, wobei mindestens ein Stickstoffatom basische Eigenschaften hat;

(Dd) Carboxylgruppen oder deren Alkalimetall- oder Erdalkalimetallsalzen;

(De) Sulfonsäuregruppen oder deren Alkalimetall- oder Erdalkalimetallsalzen;

(Df) Polyoxy-$C_2$- bis $C_4$-alkylengruppierungen, die durch Hydroxylgruppen, Mono-oder Polyaminogruppen, wobei mindestens ein Stickstoffatom basische Eigenschaften hat, oder durch Carbamatgruppen terminiert sind;

(Dg) Carbonsäureestergruppen;

(Dh) aus Bernsteinsäureanhydrid abgeleiteten Gruppierungen mit Hydroxy- und/oder Amino- und/oder Amido- und/oder Imidogruppen; und/oder

(Di) durch Mannich-Umsetzung von substituierten Phenolen mit Aldehyden und Mono- oder Polyaminen erzeugten Gruppierungen.

Der hydrophobe Kohlenwasserstoffrest in den obigen Detergenz-Additiven, welcher für die ausreichende Löslichkeit im Kraftstoff sorgt, hat ein zahlengemitteltes Molekulargewicht ($M_n$) von 85 bis 20.000, vorzugsweise von 113 bis 10.000, besonders bevorzugt von 300 bis 5.000, stärker bevorzugt von 300 bis 3.000, noch stärker bevorzugt von 500 bis 2.500 und insbesondere von 700 bis 2.500, vor allem von 800 bis 1500. Als typische hydrophobe Kohlenwasserstoffreste, kommen insbesondere Polypropenyl-, Polybutenyl- und Polyisobutenylreste mit einem zahlenmittleren Molekulargewicht $M_n$ von vorzugsweise jeweils 300 bis 5.000, besonders bevorzugt 300 bis 3.000, stärker bevorzugt 500 bis 2.500 noch stärker bevorzugt 700 bis 2.500 und insbesondere 800 bis 1.500 in Betracht.

Als Beispiele für obige Gruppen von Detergenz-Additiven seien die folgenden genannt:
Mono- oder Polyaminogruppen (Da) enthaltende Additive sind vorzugsweise Polyalkenmono- oder Polyalkenpolyamine auf Basis von Polypropen oder von hochreaktivem (d.h. mit überwiegend endständigen Doppelbindungen) oder konventionellem (d.h. mit überwiegend mittenständigen Doppelbindungen) Polybuten oder Polyisobuten mit $M_n$ = 300 bis 5000, besonders bevorzugt 500 bis 2500 und insbesondere 700 bis 2500. Derartige Additive auf Basis von hochreaktivem Polyisobuten, welche aus dem Polyisobuten, das bis zu 20 Gew.-% n-Buten-Einheiten enthalten kann, durch Hydroformylierung und reduktive Aminierung mit Ammoniak, Monoaminen oder Polyaminen wie Dimethylaminopropylamin, Ethylendiamin, Diethylentriamin, Triethylentetramin oder Tetraethylenpentamin hergestellt werden können, sind insbesondere aus der EP-A 244 616 bekannt. Geht man bei der Herstellung der Additive von Polybuten oder Polyisobuten mit überwiegend mittenständigen Doppelbindungen (meist in der $\beta$- und $\gamma$-Position) aus, bietet sich der Herstellweg durch Chlorierung und anschließende Aminierung oder durch Oxidation der Doppelbindung mit Luft oder Ozon zur Carbonyl- oder Carboxylverbindung und anschließende Aminierung unter reduktiven (hydrierenden) Bedingungen an. Zur Aminierung können hier Amine, wie z. B. Ammoniak, Monoamine oder die oben genannten Polyamine, eingesetzt werden. Entsprechende Additive auf Basis von Polypropen sind insbesondere in der WO-A 94/24231 beschrieben.

Weitere besondere Monoaminogruppen (Da) enthaltende Additive sind die Hydrierungsprodukte der Umsetzungsprodukte aus Polyisobutenen mit einem mittleren Polymerisationsgrad P = 5 bis 100 mit Stickoxiden oder Gemischen aus Stickoxiden und Sauerstoff, wie sie insbesondere in der WO-A 97/03946 beschrieben sind.

Weitere besondere Monoaminogruppen (Da) enthaltende Additive sind die aus Polyisobutenepoxiden durch Umsetzung mit Aminen und nachfolgender Dehydratisierung und Reduktion der Aminoalkohole erhältlichen Verbindungen, wie sie insbesondere in der DE-A 196 20 262 beschrieben sind.

Nitrogruppen (Db), gegebenenfalls in Kombination mit Hydroxylgruppen, enthaltende Additive sind vorzugsweise Umsetzungsprodukte aus Polyisobutenen des mittleren Polymerisationsgrades P = 5 bis 100 oder 10 bis 100 mit Stickoxiden oder Gemischen aus Stickoxiden und Sauerstoff, wie sie insbesondere in der WO-A96/03367 und in der WO-A 96/03479 beschrieben sind. Diese Umsetzungsprodukte stellen in der Regel Mischungen aus reinen Nitropolyisobutenen (z. B. $\alpha$, $\beta$ -Dinitropolyisobuten) und gemischten Hydroxynitropolyisobutenen (z. B. $\alpha$ -Nitro-$\beta$ -hydroxypolyisobuten) dar.

Hydroxylgruppen in Kombination mit Mono- oder Polyaminogruppen (Dc) enthaltende Additive sind insbesondere Umsetzungsprodukte von Polyisobutenepoxiden, erhältlich aus vorzugsweise überwiegend endständige Doppel-

bindungen aufweisendem Polyisobuten mit $M_n$ = 300 bis 5000 mit Ammoniak, Mono- oder Polyaminen, wie sie insbesondere in der EP-A 476 485 beschrieben sind.

Carboxylgruppen oder deren Alkalimetall- oder Erdalkalimetallsalze (Dd) enthaltende Additive sind vorzugsweise Copolymere von $C_2$- bis $C_{40}$-Olefinen mit Maleinsäureanhydrid mit einer Gesamt-Molmasse von 500 bis 20.000, deren Carboxylgruppen ganz oder teilweise zu den Alkalimetall- oder Erdalkalimetallsalzen und ein verbleibender Rest der Carboxylgruppen mit Alkoholen oder Aminen umgesetzt sind. Solche Additive sind insbesondere aus der EP-A 307 815 bekannt. Derartige Additive dienen hauptsächlich zur Verhinderung von Ventilsitzverschleiß und können, wie in der WO-A 87/01126 beschrieben, mit Vorteil in Kombination mit üblichen Kraftstoffdetergenzien wie Poly(iso)-butenaminen oder Polyetheraminen eingesetzt werden.

Sulfonsäuregruppen oder deren Alkalimetall- oder Erdalkalimetallsalze (De) enthaltende Additive sind vorzugsweise Alkalimetall- oder Erdalkalimetallsalze eines Sulfobernsteinsäurealkylesters, wie er insbesondere in der EP-A 639 632 beschrieben ist. Derartige Additive dienen hauptsächlich zur Verhinderung von Ventilsitzverschleiß und können mit Vorteil in Kombination mit üblichen Kraftstoffdetergenzien wie Poly(iso)buten-aminen oder Polyetheraminen eingesetzt werden.

Polyoxy-$C_2$-$C_4$-alkylengruppierungen (Df) enthaltende Additive sind vorzugsweise Polyether oder Polyetheramine, welche durch Umsetzung von $C_2$- bis $C_{60}$-Alkanolen, $C_6$-bis $C_{30}$-Alkandiolen, Mono- oder Di-$C_2$- bis $C_{30}$-alkylaminen, $C_1$- bis $C_{30}$-Alkylcyclo-hexanolen oder $C_1$- bis $C_{30}$-Alkylphenolen mit 1 bis 30 mol Ethylenoxid und/oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und US-A 4 877 416 beschrieben. Im Falle von Polyethern erfüllen solche Produkte auch Trägeröleigenschaften. Typische Beispiele hierfür sind Tridecanol- oder Isotridecanolbutoxylate, Isononylphenol-butoxylate sowie Polyisobutenolbutoxylate und -propoxylate sowie die entsprechenden Umsetzungsprodukte mit Ammoniak.

Carbonsäureestergruppen (Dg) enthaltende Additive sind vorzugsweise Ester aus Mono-, Di- oder Tricarbonsäuren mit langkettigen Alkanolen oder Polyolen, insbesondere solche mit einer Mindestviskosität von 2 $mm^2/s$ bei 100 °C, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 C-Atomen. Typische Vertreter der Ester sind Adipate, Phthalate, iso-Phthalate, Terephthalate und Trimellitate des iso-Octanols, iso-Nonanols, iso-Decanols und des iso-Tridecanols. Derartige Produkte erfüllen auch Trägeröleigenschaften.

Aus Bernsteinsäureanhydrid abgeleitete Gruppierungen mit Hydroxy- und/oder Amino-und/oder Amido- und/oder insbesondere Imidogruppen (Dh) enthaltende Additive sind vorzugsweise entsprechende Derivate von Alkyl- oder Alkenyl-substituiertem Bernsteinsäureanhydrid und insbesondere die entsprechenden Derivate von Polyisobutenylbernsteinsäureanhydrid, welche durch Umsetzung von konventionellem oder hochreaktivem Polyisobuten mit $M_n$ = vorzugsweise 300 bis 5000, besonders bevorzugt 300 bis 3000, stärker bevorzugt 500 bis 2500, noch stärker bevorzugt 700 bis 2500 und insbesondere 800 bis 1500, mit Maleinsäureanhydrid auf thermischem Weg in einer En-Reaktion oder über das chlorierte Polyisobuten erhältlich sind. Bei den Gruppierungen mit Hydroxy- und/oder Amino- und/oder Amido- und/oder Imidogruppen handelt es sich beispielsweise um Carbonsäuregruppen, Säureamide von Monoaminen, Säureamide von Di- oder Polyaminen, die neben der Amidfunktion noch freie Amingruppen aufweisen, Bernsteinsäurederivate mit einer Säure- und einer Amidfunktion, Carbonsäureimide mit Monoaminen, Carbonsäureimide mit Di- oder Polyaminen, die neben der Imidfunktion noch freie Amingruppen aufweisen, oder Diimide, die durch die Umsetzung von Di- oder Polyaminen mit zwei Bernsteinsäurederivaten gebildet werden. Beim Vorliegen von Imidogruppierungen D(h) wird das weitere Detergenz-Additiv im Sinne der vorliegenden Erfindung jedoch nur bis maximal 100 % der Gewichtsmenge an Verbindungen mit Betainstruktur eingesetzt. Derartige Kraftstoffadditive sind allgemein bekannt und beispielsweise in den Dokumenten (1) und (2) beschrieben. Bevorzugt handelt es sich um die Umsetzungsprodukte von Alkyl- oder Alkenylsubstituierten Bernsteinsäuren oder Derivaten davon mit Aminen und besonders bevorzugt um die Umsetzungsprodukte von Polyisobutenyl-substituierten Bernsteinsäuren oder Derivaten davon mit Aminen. Von besonderem Interesse sind hierbei Umsetzungsprodukte mit aliphatischen Polyaminen (Polyalkylenimine) wie insbesondere Ethylendiamin, Diethylentriamin, Triethylentetramin, Tetraethylenpentamin, Pentaethylenhexamin und Hexaethylenheptamin, welche eine Imidstruktur aufweisen.

Durch Mannich-Umsetzung von substituierten Phenolen mit Aldehyden und Mono- oder Polyaminen erzeugte Gruppierungen (Di) enthaltende Additive sind vorzugsweise Umsetzungsprodukte von Polyisobuten-substituierten Phenolen mit Formaldehyd und Mono- oder Polyaminen wie Ethylendiamin, Diethylentriamin, Triethylentetramin, Tetraethylenpentamin oder Dimethylaminopropylamin. Die Polyisobutenyl-substituierten Phenole können aus konventionellem oder hochreaktivem Polyisobuten mit $M_n$ = 300 bis 5000 stammen. Derartige "Polyisobuten-Mannichbasen" sind insbesondere in der EP-A 831 141 beschrieben.

Dem Kraftstoff können ein oder mehrere der genannten Detergenz-Additive in solch einer Menge zugegeben werden, dass die Dosierrate an diesen Detergenz-Additiven vozugsweise 25 bis 2500 Gew.-ppm, insbesondere 75 bis

1500 Gew.-ppm, vor allem 150 bis 1000 Gew.-ppm, beträgt.

B2) Trägeröle

Mitverwendete Trägeröle können mineralischer oder synthetischer Natur sein. Geeignete mineralische Trägeröle sind bei der Erdölverarbeitung anfallende Fraktionen, wie Brightstock oder Grundöle mit Viskositäten wie beispielsweise aus der Klasse SN 500 bis 2000, aber auch aromatische Kohlenwasserstoffe, paraffinische Kohlenwasserstoffe und Alkoxyalkanole. Brauchbar ist ebenfalls eine als "hydrocrack oil" bekannte und bei der Raffination von Mineralöl anfallende Fraktion (Vakuumdestillatschnitt mit einem Siedebereich von etwa 360 bis 500 °C, erhältlich aus unter Hochdruck katalytisch hydriertem und isomerisiertem sowie entparaffiniertem natürlichen Mineralöl). Ebenfalls geeignet sind Mischungen oben genannter mineralischer Trägeröle.

Beispiele für geeignete synthetische Trägeröle sind Polyolefine (Polyalphaolefine oder Polyinternalolefine), (Poly)ester, Poly)alkoxylate, Polyether, aliphatische Polyetheramine, alkylphenolgestartete Polyether, alkylphenolgestartete Polyetheramine und Carbonsäureester langkettiger Alkanole.

Beispiele für geeignete Polyolefine sind Olefinpolymerisate mit $M_n$ = 400 bis 1800, vor allem auf Polybuten- oder Polyisobuten-Basis (hydriert oder nicht hydriert).

Beispiele für geeignete Polyether oder Polyetheramine sind vorzugsweise Polyoxy-$C_2$-bis $C_4$-alkylengruppierungen enthaltende Verbindungen, welche durch Umsetzung von $C_2$- bis $C_{60}$-Alkanolen, $C_6$- bis $C_{30}$-Alkandiolen, Mono- oder Di-$C_2$- bis $C_{30}$-alkylaminen, $C_1$- bis $C_{30}$-Alkyl-cyclohexanolen oder $C_1$- bis $C_{30}$-Alkylphenolen mit 1 bis 30 mol Ethylenoxid und/ oder Propylenoxid und/oder Butylenoxid pro Hydroxylgruppe oder Aminogruppe und, im Falle der Polyetheramine, durch anschließende reduktive Aminierung mit Ammoniak, Monoaminen oder Polyaminen erhältlich sind. Derartige Produkte werden insbesondere in der EP-A 310 875, EP-A 356 725, EP-A 700 985 und der US-A 4,877,416 beschrieben. Beispielsweise können als Polyetheramine Poly-$C_2$- bis $C_6$-Alkylenoxidamine oder funktionelle Derivate davon verwendet werden. Typische Beispiele hierfür sind Tridecanol- oder Isotridecanolbutoxylate, Isononylphenolbutoxylate sowie Polyisobutenolbutoxylate und -propoxylate sowie die entsprechenden Umsetzungsprodukte mit Ammoniak.

Beispiele für Carbonsäureester langkettiger Alkanole sind insbesondere Ester aus Mono-, Di- oder Tricarbonsäuren mit langkettigen Alkanolen oder Polyolen, wie sie insbesondere in der DE-A 38 38 918 beschrieben sind. Als Mono-, Di- oder Tricarbonsäuren können aliphatische oder aromatische Säuren eingesetzt werden, als Esteralkohole bzw. -polyole eignen sich vor allem langkettige Vertreter mit beispielsweise 6 bis 24 Kohlenstoffatomen. Typische Vertreter der Ester sind Adipate, Phthalate, iso-Phthalate, Terephthalate und Trimellitate des Isooctanols, Isononanols, Isodecanols und des Isotridecanols, z. B. Di-(n- oder Isotridecyl)phthalat.

Weitere geeignete Trägerölsysteme sind beispielsweise in der DE-A 38 26 608, DE-A 41 42 241, DE-A 43 09 074, EP-A 452 328 und der EP-A 548 617 beschrieben.

Beispiele für besonders geeignete synthetische Trägeröle sind alkoholgestartete Polyether mit etwa 5 bis 35, vorzugsweise etwa 5 bis 30, besonders bevorzugt 10 bis 30 und insbesondere 15 bis 30 $C_3$- bis $C_6$-Alkylenoxideinheiten, z. B. Propylenoxid-, n-Butylenoxid- und Isobutylenoxid-Einheiten oder Gemischen davon, pro Alkoholmolekül. Nichtlimitierende Beispiele für geeignete Starteralkohole sind langkettige Alkanole oder mit langkettigem Alkyl-substituierte Phenole, wobei der langkettige Alkylrest insbesondere für einen geradkettigen oder verzweigten $C_6$- bis $C_{18}$-Alkylrest steht. Als besondere Beispiele sind zu nennen Tridecanol und Nonylphenol. Besonders bevorzugte alkoholgestartete Polyether sind die Umsetzungsprodukte (Polyveretherungsprodukte) von einwertigen aliphatischen $C_6$- bis $C_{18}$-Alkoholen mit $C_3$- bis $C_6$-Alkylenoxiden. Beispiele für einwertige aliphatische $C_6$-$C_{18}$-Alkohole sind Hexanol, Heptanol, Octanol, 2-Ethylhexanol, Nonylalkohol, Decanol, 3-Propylheptanol, Undecanol, Dodecanol, Tridecanol, Tetradecanol, Pentadecanol, Hexadecanol, Octadecanol und deren Konstitutions- und Stellungsisomere. Die Alkohole können sowohl in Form der reinen Isomere als auch in Form technischer Gemische eingesetzt werden. Ein besonders bevorzugter Alkohol ist Tridecanol. Beispiele für $C_3$- bis $C_6$-Alkylenoxide sind Propylenoxid, wie 1,2-Propylenoxid, Butylenoxid, wie 1,2-Butylenoxid, 2,3-Butylenoxid, Isobutylenoxid oder Tetrahydrofuran, Pentylenoxid und Hexylenoxid. Besonders bevorzugt sind hierunter $C_3$- bis $C_4$-Alkylenoxide, d.h. Propylenoxid wie 1,2-Propylenoxid und Butylenoxid wie 1,2-Butylenoxid, 2,3-Butylenoxid und Isobutylenoxid. Speziell verwendet man Butylenoxid.

Weitere geeignete synthetische Trägeröle sind alkoxylierte Alkylphenole, wie sie in der DE-A 10 102 913 beschrieben sind.

Besondere Trägeröle sind synthetische Trägeröle, wobei die zuvor beschriebenen alkoholgestarteten Polyether besonders bevorzugt sind.

Das Trägeröl bzw. das Gemisch verschiedener Trägeröle wird dem Kraftstoff in einer Menge von vorzugsweise 1 bis 1000 Gew.-ppm, besonders bevorzugt von 10 bis 500 Gew.-ppm und insbesondere von 20 bis 100 Gew.-ppm zugesetzt.

B3) Kaltfließverbesserer

Geeignete Kaltfließverbesserer sind im Prinzip alle organischen Verbindungen, welche in der Lage sind, das Fließverhalten von Mitteldestillat-Kraftstoffen bzw. Dieselkraftstoffen in der Kälte zu verbessern. Zweckmäßigerweise müssen sie eine ausreichende Öllöslichkeit aufweisen. Insbesondere kommen hierfür die üblicherweise bei Mitteldestillaten aus fossilem Ursprung, also bei üblichen mineralischen Dieselkraftstoffen, eingesetzten Kaltfließverbesserer (" middle distillate flow improvers" , " MDFI") in Betracht. Jedoch können auch organische Verbindungen verwendet werden, die beim Einsatz in üblichen Dieselkraftstoffen zum Teil oder überwiegend die Eigenschaften eines Wax Anti-Settling Additivs ("WASA") aufweisen. Auch können sie zum Teil oder überwiegend als Nukleatoren wirken. Es können aber auch Mischungen aus als MDFI wirksamen und/oder als WASA wirksamen und/oder als Nukleatoren wirksamen organischen Verbindungen eingesetzt werden.

Typischerweise wird der Kaltfließverbesserer ausgewählt aus:

(K1) Copolymeren eines $C_2$- bis $C_{40}$-Olefins mit wenigstens einem weiteren ethylenisch ungesättigten Monomer;
(K2) Kammpolymeren;
(K3) Polyoxyalkylenen;
(K4) polaren Stickstoffverbindungen;
(K5) Sulfocarbonsäuren oder Sulfonsäuren oder deren Derivaten; und
(K6) Poly(meth)acrylsäureestern.

Es können sowohl Mischungen verschiedener Vertreter aus einer der jeweiligen Klassen (K1) bis (K6) als auch Mischungen von Vertretern aus verschiedenen Klassen (K1) bis (K6) eingesetzt werden.

Geeignete $C_2$- bis $C_{40}$-Olefin-Monomere für die Copolymeren der Klasse (K1) sind beispielsweise solche mit 2 bis 20, insbesondere 2 bis10 Kohlenstoffatomen sowie mit 1 bis 3, vorzugsweise mit 1 oder 2, insbesondere mit einer Kohlenstoff-Kohlenstoff-Doppelbindung. Im zuletzt genannten Fall kann die Kohlenstoff-Kohlenstoff-Doppelbindung sowohl terminal ($\alpha$-Olefine) als auch intern angeordnet sein kann. Bevorzugt sind jedoch $\alpha$ -Olefine, besonders bevorzugt $\alpha$ -Olefine mit 2 bis 6 Kohlenstoffatomen, beispielsweise Propen, 1-Buten, 1-Penten, 1-Hexen und vor allem Ethylen.

Bei den Copolymeren der Klasse (K1) ist das wenigstens eine weitere ethylenisch ungesättigte Monomer vorzugsweise ausgewählt unter Carbonsäurealkenylestern, (Meth)Acrylsäureestern und weiteren Olefinen.

Werden weitere Olefine mit einpolymerisiert, sind dies vorzugsweise höhermolekulare als das oben genannte $C_2$- bis $C_{40}$-Olefin-Basismonomere. Setzt man beispielsweise als Olefin-Basismonomer Ethylen oder Propen ein, eignen sich als weitere Olefine insbesondere $C_{10}$- bis $C_{40}$-$\alpha$ -Olefine. Weitere Olefine werden in den meisten Fällen nur dann mit einpolymerisiert, wenn auch Monomere mit Carbonsäureester-Funktionen eingesetzt werden.

Geeignete (Meth)Acrylsäureester sind beispielsweise Ester der (Meth)Acrylsäure mit $C_1$- bis $C_{20}$-Alkanolen, insbesondere $C_1$- bis $C_{10}$-Alkanolen, vor allem mit Methanol, Ethanol, Propanol, Isopropanol, n-Butanol, sec.-Butanol, Isobutanol, tert.-Butanol, Pentanol, Hexanol, Heptanol, Octanol, 2-Ethylhexanol, Nonanol und Decanol sowie Strukturisomeren hiervon.

Geeignete Carbonsäurealkenylester sind beispielsweise $C_2$- bis $C_{14}$-Alkenylester, z.B. die Vinyl- und Propenylester, von Carbonsäuren mit 2 bis 21 Kohlenstoffatomen, deren Kohlenwasserstoffrest linear oder verzweigt sein kann. Bevorzugt sind hierunter die Vinylester. Unter den Carbonsäuren mit verzweigtem Kohlenwasserstoffrest sind solche bevorzugt, deren Verzweigung sich in der $\alpha$-Position zur Carboxylgruppe befindet, wobei das $\alpha$-Kohlenstoffatom besonders bevorzugt tertiär ist, d. h. die Carbonsäure eine sogenannte Neocarbonsäure ist. Vorzugsweise ist der Kohlenwasserstoffrest der Carbonsäure jedoch linear.

Beispiele für geeignete Carbonsäurealkenylester sind Vinylacetat, Vinylpropionat, Vinylbutyrat, Vinyl-2-ethylhexanoat, Neopentansäurevinylester, Hexansäurevinylester, Neononansäurevinylester, Neodecansäurevinylester und die entsprechenden Propenyl-ester, wobei die Vinylester bevorzugt sind. Ein besonders bevorzugter Carbonsäurealkenylester ist Vinylacetat; typische hieraus resultierende Copolymere der Gruppe (K1) sind die mit am häufigsten eingesetzten Ethylen-Vinylacetat-Copolymere ("EVA").

Besonders vorteilhaft einsetzbare Ethylen-Vinylacetat-Copolymere und ihre Herstellung sind in der WO 99/29748 beschrieben.

Als Copolymere der Klasse (K1) sind auch solche geeignet, die zwei oder mehrere voneinander verschiedene Carbonsäurealkenylester einpolymerisiert enthalten, wobei diese sich in der Alkenylfunktion und/oder in der Carbonsäuregruppe unterscheiden. Ebenfalls geeignet sind Copolymere, die neben dem/den Carbonsäurealkenylester(n) wenigstens ein Olefin und/oder wenigstens ein (Meth)Acrylsäureester einpolymerisiert enthalten.

Auch Terpolymere aus einem $C_2$- bis $C_{40}$-$\alpha$ -Olefin, einem $C_1$- bis $C_{20}$-Alkylester einer ethylenisch ungesättigten Monocarbonsäure mit 3 bis 15 Kohlenstoffatomen und einem $C_2$- bis $C_{14}$-Alkenylester einer gesättigten Monocarbonsäure mit 2 bis 21 Kohlenstoffatomen sind als Copolymere der Klasse (K1) geeignet. Derartige Terpolymere sind in der WO 2005/054314 beschrieben. Ein typisches derartiges Terpolymer ist aus Ethylen, Acrylsäure-2-ethylhexylester und

Vinylacetat aufgebaut.

Das wenigstens eine oder die weiteren ethylenisch ungesättigten Monomeren sind in den Copolymeren der Klasse (K1) in einer Menge von vorzugsweise 1 bis 50 Gew.-%, insbesondere von 10 bis 45 Gew.-% und vor allem von 20 bis 40 Gew.-%, bezogen auf das Gesamtcopolymer, einpolymerisiert. Der gewichtsmäßige Hauptanteil der Monomereinheiten in den Copolymeren der Klasse (K1) stammt somit in der Regel aus den $C_2$- bis $C_{40}$-Basis-Olefinen.

Die Copolymere der Klasse (K1) weisen vorzugsweise ein zahlenmittleres Molekulargewicht $M_n$ von 1000 bis 20.000, besonders bevorzugt von 1000 bis 10.000 und insbesondere von 1000 bis 8000 auf.

Typische Kammpolymere der Komponente (K2) sind beispielsweise durch die Copolymerisation von Maleinsäureanhydrid oder Fumarsäure mit einem anderen ethylenisch ungesättigten Monomer, beispielsweise mit einem $\alpha$ -Olefin oder einem ungesättigten Ester wie Vinylacetat, und anschließende Veresterung der Anhydrid- bzw. Säurefunktion mit einem Alkohol mit wenigstens 10 Kohlenstoffatomen erhältlich. Weitere geeignete Kammpolymere sind Copolymere von $\alpha$ -Olefinen und veresterten Comonomeren, beispielsweise veresterte Copolymere von Styrol und Maleinsäureanhydrid oder veresterte Copolymere von Styrol und Fumarsäure. Geeignete Kammpolymere können auch Polyfumarate oder Polymaleinate sein. Außerdem sind Homo- und Copolymere von Vinylethern geeignete Kammpolymere. Als Komponente der Klasse (K2) geeignete Kammpolymere sind beispielsweise auch solche, die in der WO 2004/035715 und in "Comb-Like Polymers. Structure and Properties" , N. A. Plate und V. P. Shibaev, J. Poly. Sci. Macromolecular Revs. 8, Seiten 117 bis 253 (1974)" beschrieben sind. Auch Gemische von Kammpolymeren sind geeignet.

Als Komponente der Klasse (K3) geeignete Polyoxyalkylene sind beispielsweise Polyoxyalkylenester, Polyoxyalkylenether, gemischte Polyoxyalkylenesterether und Gemische davon. Bevorzugt enthalten diese Polyoxyalkylenverbindungen wenigstens eine, vorzugsweise wenigstens zwei lineare Alkylgruppen mit jeweils 10 bis 30 Kohlenstoffatomen und eine Polyoxyalkylengruppe mit einem zahlenmittleren Molekulargewicht von bis zu 5000. Derartige Polyoxyalkylenverbindungen sind beispielsweise in der EP-A 061 895 sowie in der US 4 491 455 beschrieben. Besondere Polyoxyalkylenverbindungen basieren auf Polyethylenglykolen und Polypropylenglykolen mit einem zahlenmittleren Molekulargewicht von 100 bis 5000. Weiterhin sind Polyoxyalkylenmono- und -diester von Fettsäuren mit 10 bis 30 Kohlenstoffatomen wie Stearinsäure oder Behensäure geeignet.

Als Komponente der Klasse (K4) geeignete polare Stickstoffverbindungen können sowohl ionischer als auch nicht ionischer Natur sein und besitzen vorzugsweise wenigstens einen, insbesondere wenigstens zwei Substituenten in Form eines tertiären Stickstoffatoms der allgemeinen Formel $>NR^7$, worin $R^7$ für einen $C_8$- bis $C_{40}$-Kohlenwasserstoffrest steht. Die Stickstoffsubstituenten können auch quaternisiert, das heißt in kationischer Form, vorliegen. Beispiele für solche Stickstoffverbindungen sind Ammoniumsalze und/oder Amide, die durch die Umsetzung wenigstens eines mit wenigstens einem Kohlenwasserstoffrest substituierten Amins mit einer Carbonsäure mit 1 bis 4 Carboxylgruppen bzw. mit einem geeignetem Derivat davon erhältlich sind. Vorzugsweise enthalten die Amine wenigstens einen linearen $C_8$- bis $C_{40}$-Alkylrest. Zur Herstellung der genannten polaren Stickstoffverbindungen geeignete primäre Amine sind beispielsweise Octylamin, Nonylamin, Decylamin, Undecylamin, Dodecylamin, Tetradecylamin und die höheren linearen Homologen, hierzu geeignete sekundäre Amine sind beispielsweise Dioctadecylamin und Methylbehenylamin. Geeignet sind hierzu auch Amingemische, insbesondere großtechnisch zugängliche Amingemische wie Fettamine oder hydrierte Tallamine, wie sie beispielsweise in Ullmanns Encyclopedia of Industrial Chemistry, 6. Auflage, im Kapitel " Amines, aliphatic" beschrieben werden. Für die Umsetzung geeignete Säuren sind beispielsweise Cyclohexan-1,2-dicarbonsäure, Cyclohexen-1,2-dicarbonsäure, Cyclopentan-1,2-dicarbonsäure, Naphthalindicarbonsäure, Phthalsäure, Isophthalsäure, Terephthalsäure und mit langkettigen Kohlenwasserstoffresten substituierte Bernsteinsäuren.

Insbesondere ist die Komponente der Klasse (K4) ein öllösliches Umsetzungsprodukt aus mindestens eine tertiäre Aminogruppe aufweisenden Poly($C_2$- bis $C_{20}$-Carbon-säuren) mit primären oder sekundären Aminen. Die diesem Umsetzungsprodukt zugrundeliegenden mindestens eine tertiäre Aminogruppe aufweisenden Poly($C_2$- bis $C_{20}$-Carbonsäuren) enthalten vorzugsweise mindestens 3 Carboxylgruppen, insbesondere 3 bis 12, vor allem 3 bis 5 Carboxylgruppen. Die Carbonsäure-Einheiten in den Polycarbonsäuren weisen vorzugsweise 2 bis 10 Kohlenstoffatome auf, insbesondere sind es Essigsäure-Einheiten. Die Carbonsäure-Einheiten sind in geeigneter Weise zu den Polycarbonsäuren verknüpft, meist über ein oder mehrere Kohlenstoff- und/oder Stickstoffatome. Vorzugsweise sind sie an tertiäre Stickstoffatome angebunden, die im Falle mehrerer Stickstoffatome über Kohlenwasserstoffketten verbunden sind.

Vorzugsweise ist die Komponente der Klasse (K4) ein öllösliches Umsetzungsprodukt auf Basis von mindestens eine tertiäre Aminogruppe aufweisenden Poly($C_2$- bis $C_{20}$-Carbonsäuren) der allgemeinen Formel IIa oder IIb

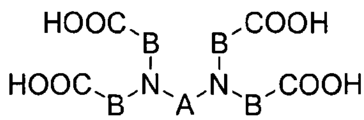

(IIa)

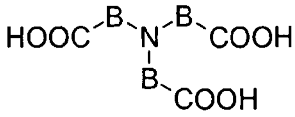

(IIb)

in denen die Variable A eine geradkettige oder verzweigte $C_2$- bis $C_6$-Alkylengruppe oder die Gruppierung der Formel III

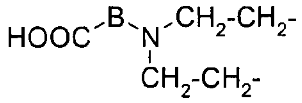

(III)

darstellt und die Variable B eine $C_1$- bis $C_{19}$-Alkylengruppe bezeichnet. Die Verbindungen der allgemeinen Formel IIa und IIb weisen insbesondere die Eigenschaften eines WASA auf.

Weiterhin ist das bevorzugte öllösliche Umsetzungsprodukt der Komponente (K4), insbesondere das der allgemeinen Formel IIa oder IIb, ein Amid, ein Amidammoniumsalz oder ein Ammoniumsalz, in dem keine, eine oder mehrere Carbonsäuregruppen in Amidgruppen übergeführt sind.

Geradkettige oder verzweigte $C_2$- bis $C_6$-Alkylengruppen der Variablen A sind beispielsweise 1,1-Ethylen, 1,2-Propylen, 1,3-Propylen, 1,2-Butylen, 1,3-Butylen, 1,4-Butylen, 2-Methyl-1,3-propylen, 1,5-Pentylen, 2-Methyl-1,4-butylen, 2,2-Dimethyl-1,3-propylen, 1,6-Hexylen (Hexamethylen) und insbesondere 1,2-Ethylen. Vorzugsweise umfasst die Variable A 2 bis 4, insbesondere 2 oder 3 Kohlenstoffatome.

$C_1$- bis $C_{19}$-Alkylengruppen der Variablen B sind vor beispielsweise 1,2-Ethylen, 1,3-Propylen, 1,4-Butylen, Hexamethylen, Octamethylen, Decamethylen, Dodecamethylen, Tetradecamethylen, Hexadecamethylen, Octadecamethylen, Nonadecamethylen und insbesondere Methylen. Vorzugsweise umfasst die Variable B 1 bis 10, insbesondere 1 bis 4 Kohlenstoffatome.

Die primären und sekundären Amine als Umsetzungspartner für die Polycarbonsäuren zur Bildung der Komponente (K4) sind üblicherweise Monoamine, insbesondere aliphatische Monoamine. Diese primären und sekundären Amine können aus einer Vielzahl von Aminen ausgewählt sein, die - gegebenenfalls miteinander verbundene - Kohlenwasserstoffreste tragen.

Meist sind diese den öllöslichen Umsetzungsprodukten der Komponente (K4) zugrundeliegenden Amine sekundären Amine und weisen die allgemeine Formel $HN(R^8)_2$ auf, in der die beiden Variablen $R^8$ unabhängig voneinander jeweils geradkettige oder verzweigte $C_{10}$- bis $C_{30}$-Alkylreste, insbesondere $C_{14}$- bis $C_{24}$-Alkylreste bedeuten. Diese längerkettigen Alkylreste sind vorzugsweise geradkettig oder nur in geringem Grade verzweigt. In der Regel leiten sich die genannten sekundären Amine hinsichtlich ihrer längerkettigen Alkylreste von natürlich vorkommenden Fettsäuren bzw. von deren Derivaten ab. Vorzugsweise sind die beiden Reste $R^8$ gleich.

Die genannten sekundären Amine können mittels Amidstrukturen oder in Form der Ammoniumsalze an die Polycarbonsäuren gebunden sein, auch kann nur ein Teil als Amidstrukturen und ein anderer Teil als Ammoniumsalze vorliegen. Vorzugsweise liegen nur wenige oder keine freien Säuregruppen vor. Vorzugsweise liegen die öllöslichen Umsetzungsprodukte der Komponente (K4) vollständig in Form der Amidstrukturen vor.

Typische Beispiele für derartige Komponenten (K4) sind Umsetzungsprodukte der Nitrilotriessigsäure, der Ethylendiamintetraessigsäure oder der Propylen-1,2-diamintetraessigsäure mit jeweils 0,5 bis 1,5 Mol pro Carboxylgruppe, insbesondere 0,8 bis 1,2 Mol pro Carboxylgruppe, Dioleylamin, Dipalmitinamin, Dikokosfettamin, Distearylamin, Dibehenylamin oder insbesondere Ditalgfettamin. Eine besonders bevorzugte Komponente (K4) ist das Umsetzungsprodukt aus 1 Mol Ethylendiamintetraessigsäure und 4 Mol hydriertem Ditalgfettamin.

Als weitere typische Beispiele für die Komponente (K4) seien die N,N-Dialkylammoniumsalze von 2-N' ,N' -Dialkylamidobenzoaten, beispielsweise das Reaktionsprodukt aus 1 Mol Phthalsäureanhydrid und 2 Mol Ditalgfettamin, wobei letzteres hydriert oder nicht hydriert sein kann, und das Reaktionsprodukt von 1 Mol eines Alkenylspirobislactons mit 2 Mol eines Dialkylamins, beispielsweise Ditalgfettamin und/oder Talgfettamin, wobei die beiden letzteren hydriert oder nicht hydriert sein können, genannt.

Weitere typische Strukturtypen für die Komponente der Klasse (K4) sind cyclische Verbindungen mit tertiären Aminogruppen oder Kondensate langkettiger primärer oder sekundärer Amine mit carbonsäurehaltigen Polymeren, wie sie in der WO 93/18115 beschrieben sind.

Als Kaltfließverbesserer der Komponente der Klasse (K5) geeignete Sulfocarbonsäuren, Sulfonsäuren oder deren Derivate sind beispielsweise die öllöslichen Carbonsäureamide und Carbonsäureester von ortho-Sulfobenzoesäure, in denen die Sulfonsäurefunktion als Sulfonat mit alkylsubstituierten Ammoniumkationen vorliegt, wie sie in der EP-A 261 957 beschrieben werden.

Als Kaltfließverbesserer der Komponente der Klasse (K6) geeignete Poly(meth)acrylsäureester sind sowohl Homo- als auch Copolymere von Acryl- und Methacrylsäureestern. Bevorzugt sind Copolymere von wenigstens zwei

voneinander verschiedenen (Meth)Acrylsäureestern, die sich bezüglich des einkondensierten Alkohols unterscheiden. Gegebenenfalls enthält das Copolymer noch ein weiteres, davon verschiedenes olefinisch ungesättigtes Monomer einpolymerisiert. Das gewichtsmittlere Molekulargewicht des Polymers beträgt vorzugsweise 50.000 bis 500.000. Ein besonders bevorzugtes Polymer ist ein Copolymer von Methacrylsäure und Methacrylsäureestern von gesättigten $C_{14}$- und $C_{15}$-Alkoholen, wobei die Säuregruppen mit hydriertem Tallamin neutralisiert sind. Geeignete Poly(meth)acrylsäureester sind beispielsweise in der WO 00/44857 beschrieben.

Dem Mitteldestillat-Kraftstoff bzw. Dieselkraftstoff wird der Kaltfließverbesserer bzw. das Gemisch verschiedener Kaltfließverbesserer in einer Gesamtmenge von vorzugsweise 10 bis 5000 Gew.-ppm, besonders bevorzugt von 20 bis 2000 Gew.-ppm, stärker bevorzugt von 50 bis 1000 Gew.-ppm und insbesondere von 100 bis 700 Gew.-ppm, z.B. von 200 bis 500 Gew.-ppm, zugegeben.

B4) Schmierfähigkeitsverbesserer

Geeignete Schmierfähigkeitsverbesserer (Lubricity Improver bzw. Friction Modifier) basieren üblicherweise auf Fettsäuren oder Fettsäureestern. Typische Beispiele sind Tallölfettsäure, wie beispielsweise in der WO 98/004656 beschrieben, und Glycerinmonooleat. Auch die in der US 6 743 266 B2 beschriebenen Reaktionsprodukte aus natürlichen oder synthetischen Ölen, beispielsweise Triglyceriden, und Alkanolaminen sind als solche Schmierfähigkeitsverbesserer geeignet.

B5) Korrosionsinhibitoren

Geeignete Korrosionsinhibitoren sind z.B. Bernsteinsäureester, vor allem mit Polyolen, Fettsäurederivate, z.B. Ölsäureester, oligomerisierte Fettsäuren, substituierte Ethanolamine und Produkte, die unter dem Handelsnamen RC 4801 (Rhein Chemie Mannheim, Deutschland) oder HiTEC 536 (Ethyl Corporation) vertrieben werden.

B6) Demulgatoren

Geeignete Demulgatoren sind z.B. die Alkali- oder Erdalkalisalze von Alkyl-substituierten Phenol- und Naphthalinsulfonaten und die Alkali- oder Erdalkalisalze von Fettsäuren, außerdem neutrale Verbindungen wie Alkoholalkoxylate, z.B. Alkoholethoxylate, Phenolalkoxylate, z.B. tert-Butylphenolethoxylat oder tert-Pentylphenolethoxylat, Fettsäuren, Alkylphenole, Kondensationsprodunkte von Ethylenoxid (EO) und Propylenoxid (PO), z.B. auch in Form von EO/PO-Blockcopolymeren, Polyethylenimine oder auch Polysiloxane.

B7) Dehazer

Geeignete Dehazer sind z.B. alkoxylierte Phenol-Formaldehyd-Kondensate, wie beispielsweise die unter dem Handelsnamen erhältlichen Produkte NALCO 7D07 (Nalco) und TOLAD 2683 (Petrolite).

B8) Antischaummittel

Geeignete Antischaummittel sind z.B. Polyether-modifizierte Polysiloxane, wie beispielsweise die unter dem Handelsnamen erhältlichen Produkte TEGOPREN 5851 (Goldschmidt), Q 25907 (Dow Corning) und RHODOSIL (Rhone Poulenc).

B9) Cetanzahlverbesserer

Geeignete Cetanzahlverbesserer sind z.B. aliphatische Nitrate wie 2-Ethylhexylnitrat und Cyclohexylnitrat sowie Peroxide wie Di-tert-butylperoxid.

B10) Antioxidantien

Geeignete Antioxidantien sind z.B. substituierte Phenole, wie 2,6-Di-tert.-butylphenol und 6-Di-tert.-butyl-3-methylphenol sowie Phenylendiamine wie N,N'-Di-sec.-butyl-p-phenylendiamin.

B11) Metalldeaktivatoren

Geeignete Metalldeaktivatoren sind z.B. Salicylsäurederivate wie N,N'-Disalicyliden-1,2-propandiamin.

B12) Lösungsmittel

Geeignete sind z.B. unpolare organische Lösungsmittel wie aromatische und aliphatische Kohlenwasserstoffe, beispielsweise Toluol, Xylole, "white spirit" und Produkte, die unter dem Handelsnamen SHELLSOL (Royal Dutch/Shell Group) und EXXSOL (ExxonMobil) vertrieben werden, sowie polare organische Lösungsmittel, beispielsweise Alkohole wie 2-Ethylhexanol, Decanol und Isotridecanol. Derartige Lösungsmittel gelangen meist zusammen mit den vorgenannten Additiven und Co-Additiven, die sie zur besseren Handhabung lösen oder verdünnen sollen, in den Dieselkraftstoff.

**C) Kraftstoffe**

Das erfindungsgemäße Additiv eignet sich in hervorragender Weise als Kraftstoffzusatz und kann im Prinzip in jeglichen Kraftstoffen eingesetzt werden. Es bewirkt eine ganze Reihe von vorteilhaften Effekten beim Betrieb von Verbrennungsmotoren mit Kraftstoffen. Bevorzugt wird das erfindungsgemäße quaternisierte Additiv in Mitteldestillat-Kraftstoffen, insbesondere Dieselkraftstoffen, eingesetzt.

Gegenstand der vorliegenden Erfindung sind daher auch Kraftstoffe, insbesondere Mitteldestillat-Kraftstoffe, mit einem als Zusatzstoff zur Erzielung von vorteilhaften Effekten beim Betrieb von Verbrennungsmotoren, beispielsweise von Dieselmotoren, insbesondere von direkteinspritzenden Dieselmotoren, vor allem von Dieselmotoren mit Common-Rail-Einspritzsystemen, wirksamen Gehalt an dem erfindungsgemäßen quaternisierten Additiv. Dieser wirksame Gehalt (Dosierrate) in erfindungsgemäßen Kraftstoffen liegt in der Regel bei 10 bis 5000 Gew.-ppm, vorzugsweise bei 20 bis 1500 Gew.-ppm, insbesondere bei 25 bis 1000 Gew.-ppm, vor allem bei 30 bis 750 Gew.-ppm, jeweils bezogen auf die Gesamtmenge an Kraftstoff.

Bei Mitteldestillat-Kraftstoffen wie Dieselkraftstoffen oder Heizölen handelt es sich vorzugsweise um Erdölraffinate, die üblicherweise einen Siedebereich von 100 bis 400°C haben. Dies sind meist Destillate mit einem 95%-Punkt bis zu 360°C oder auch darüber hinaus. Dies können aber auch so genannte "Ultra Low Sulfur Diesel" oder "City Diesel" sein, gekennzeichnet durch einen 95%-Punkt von beispielsweise maximal 345°C und einem Schwefelgehalt von maximal 0,005 Gew.-% oder durch einen 95%-Punkt von beispielsweise 285°C und einem Schwefelgehalt von maximal 0,001 Gew.-%. Neben den durch Raffination erhältlichen mineralischen Mitteldestillat-Kraftstoffen bzw. Dieselkraftstoffen sind auch solche, die durch Kohlevergasung oder Gasverflüssigung ["gas to liquid" (GTL)-Kraftstoffe] oder durch Biomasse-Verflüssigung ["biomass to liquid" (BTL)-Kraftstoffe] erhältlich sind, geeignet. Geeignet sind auch Mischungen der vorstehend genannten Mitteldestillat-Kraftstoffe bzw. Dieselkraftstoffe mit regenerativen Kraftstoffen, wie Biodiesel oder Bioethanol.

Die Qualitäten der Heizöle und Dieselkraftstoffe sind beispielsweise in DIN 51603 und EN 590 näher festgelegt (vgl. auch Ullmann' s Encyclopedia of Industrial Chemistry, 5. Auflage, Band A12, S. 617 ff.).

Das erfindungsgemäße quaternisierte Additiv kann neben seiner Verwendung in den oben genannten Mitteldestillat-Kraftstoffen aus fossilem, pflanzlichem oder tierischem Ursprung, die im wesentlichen Kohlenwasserstoffmischungen darstellen, auch in Mischungen aus solchen Mitteldestillaten mit Biobrennstoffölen (Biodiesel) eingesetzt werden. Derartige Mischungen werden im Sinne der vorliegenden Erfindung auch von dem Begriff " Mitteldestillat-Kraftstoff" umfasst. Sie sind handelsüblich und enthalten meist die Biobrennstofföle in untergeordneten Mengen, typischerweise in Mengen von 1 bis 30 Gew.-% insbesondere von 3 bis 10 Gew.-%, bezogen auf die Gesamtmenge aus Mitteldestillat fossilen, pflanzlichem oder tierischen Ursprungs und Biobrennstofföl.

Biobrennstofföle basieren in der Regel auf Fettsäureestern, vorzugsweise im wesentlichen auf Alkylester von Fettsäuren, die sich von pflanzlichen und/oder tierischen Ölen und/oder Fetten ableiten. Unter Alkylestern werden üblicherweise Niedrigalkylester, insbesondere $C_1$- bis $C_4$-Alkylester, verstanden, die durch Umesterung der in pflanzlichen und/oder tierischen Ölen und/oder Fetten vorkommenden Glyceride, insbesondere Triglyceride, mittels Niedrigalkoholen, beispielsweise Ethanol oder vor allem Methanol ("FAME"), erhältlich sind. Typische Niedrigalkylester auf Basis von pflanzlichen und/oder tierischen Ölen und/oder Fetten, die als Biobrennstofföl oder Komponenten hierfür Verwendung finden, sind beispielsweise Sonnenblumenmethylester, Palmölmethylester ("PME"), Sojaölmethylester ("SME") und insbesondere Rapsölmethylester ("RME").

Besonders bevorzugt handelt es sich bei den Mitteldestillat-Kraftstoffen bzw. Dieselkraftstoffen um solche mit niedrigem Schwefelgehalt, das heißt mit einem Schwefelgehalt von weniger als 0,05 Gew.-%, vorzugsweise von weniger als 0,02 Gew.-%, insbesondere von weniger als 0,005 Gew.-% und speziell von weniger als 0,001 Gew.-% Schwefel.

Als Ottokraftstoffe kommen alle handelsüblichen Ottokraftstoffzusammensetzungen in Betracht. Als typischer Vertreter soll hier der marktübliche Eurosuper Grundkraftstoff gemäß EN 228 genannt werden. Weiterhin sind auch Ottokraftstoffzusammensetzungen der Spezifikation gemäß WO 00/47698 mögliche Einsatzgebiete für die vorliegende Erfindung.

Das erfindungsgemäße quaternisierte Additiv eignet sich insbesondere als Kraftstoffzusatz in Kraftstoffzusammensetzungen, insbesondere in Dieselkraftstoffen, zur Überwindung der eingangs geschilderten Probleme bei direkteinspritzenden Dieselmotoren, vor allem bei solchen mit Common-Rail-Einspritzsystemen.

Die Erfindung wird nun anhand der folgenden Ausführungsbeispiele näher beschrieben:

## Experimenteller Teil:

### Verwendete Reagenzien:

Polyisobuten 1000: Glissopal® 1000 von BASF, Mn = 1000
Ameisensäure von BASF, CAS 64-18-6
Methyltrioctylammoniumchlorid von Aldrich, 63393-96-4
n-Heptan von Aldrich, CAS 142-82-5
Methanol von Aldrich, CAS 67-56-1
Essigsäure (99-100%) von Roth, CAS 64-19-7
N-Methylpyrrolidin von BASF, CAS 120-94-5
1-Dodecenoxid von Aldrich, CAS 2855-19-8
1-Tetradecenoxid, CAS 3234-28-4
1-Hexadecenoxid, CAS 7320-37-8
Isopropanol von Aldrich, CAS 67-63-0
Trimethylamin $NMe_3$ (>99,5% (w/w)) von BASF, CAS 75-50-3
Tallölfettsäure: Kerokorr® LA 99 C von BASF, TAN 199 mg KOH/g
N,N-Dimethylethanolamin von BASF, CAS 108-01-0
Salicylsäure von Merck, CAS 69-72-7

### A. Allgemeine Testmethoden

Diese Methoden gelten als Teil der allgemeinen Offenbarung und sind nicht auf die konkret getesteten Verbindungen beschränkt.

### Motorentest

### 1. XUD9 Test - Bestimmung der Flow Restriction

Die Durchführung erfolgt nach den Standardbestimmungen gemäß CEC F-23-1-01.

### 2. DW10 Test - Bestimmung des Leistungsverlusts durch Injektorablagerungen im Common Rail Dieselmotor

### 2.1. DW10- KC - Keep Clean Test

Der Keep Clean Test lehnt sich an die CEC Test Prozedur F-098-08 Issue 5 an. Dabei kommen der gleiche Testaufbau und Motorentyp (PEUGEOT DW10) wie in der CEC Prozedur zum Einsatz.

### Änderung und Besonderheiten:

Bei den Versuchen kamen gereinigte Injektoren zum Einsatz. Die Reinigungsdauer im Ultraschallbad in 60°C Wasser + 10% Superdecontamine (Intersciences, Brüssel) betrug 4h.

### Testlaufzeiten:

Der Testzeitraum betrug 12h ohne Abstellphasen. Der in Figur 1 dargestellte einstündige Testzyklus aus der CEC F-098-08 wurde dabei 12-mal durchfahren.

### Leistungsbestimmung:

Die Anfangsleistung P0,KC [kW] wird aus dem gemessenen Drehmoment bei 4000/min Volllast direkt nach Teststart und Warmlauf des Motors berechnet. Die Vorgehensweise ist in der Issue 5 der Testprozedur (CEC F-98-08) beschrieben. Dabei wird der gleiche Testaufbau und der Motorentyp PEUGEOT DW10 verwendet.

Die Endleistung (Pend,KC) wird im 12. Zyklus in Stufe 12, (siehe Tabelle, Figur 1) bestimmt. Auch hier ist der Betriebspunkt 4000/min Volllast. Pend,KC [kW] errechnet sich aus dem gemessenen Drehmoment.

Der Leistungsverlust im KC Test wird wie folgt berechnet:

$$\text{Powerloss,KC}\,[\%] = \left(1 - \frac{\text{Pend,KC}}{\text{P0,KC}}\right) * 100$$

### 2.2. DW10-Dirty Up - Clean Up-(DU-CU)

Der DU-CU Test lehnt sich an die CEC Test Prozedur F-098-08 Issue 5 an. Die Vorgehensweise ist in der Issue 5 der Testprozedur (CEC F-98-08) beschrieben. Dabei wird der gleiche Testaufbau und der Motorentyp PEUGEOT DW10 verwendet.

Der DU - CU Test besteht aus zwei einzelnen Tests, die hintereinander gefahren werden. Der erste Test dient zur Ablagerungsbildung (DU), der zweite zum Entfernen der Ablagerungen (CU). Nach dem DU wird der Leistungsverlust (Powerloss) bestimmt. Nach Ende des DU Laufs wird der Motor für mindestens 8 Stunden nicht betrieben und auf Umgebungstemperatur abgekühlt. Danach wird mit dem CU Kraftstoff der CU gestartet, ohne die Injektoren auszubauen und zu reinigen. Die Ablagerungen und der powerloss gehen im Idealfall im CU-Testverlauf zurück.

**Änderung und Besonderheiten:**

Gereinigte Injektoren wurden vor jedem DU Test in den Motor eingebaut. Die Reinigungsdauer im Ultraschallbad bei 60°C, Wasser + 10% Superdecontamine (Intersciences, Brüssel) betrug 4h.

**Testlaufzeiten:**

Der Testzeitraum betrug 12h für den DU und 12h für den CU. Der Motor wurde im DU und CU Test ohne Abstellphasen betrieben.

Der in Figur 1 dargestellte einstündige Testzyklus aus der CEC F-098-08 wurde dabei jeweils 12-mal durchfahren.

**Leistungsbestimmung:**

Die Anfangsleistung P0,du [kW] wird aus dem gemessenen Drehmoment bei 4000/min Volllast direkt nach Teststart und Warmlauf des Motors berechnet. Die Vorgehensweise ist ebenfalls in der Issue 5 der Testprozedur beschrieben.

Die Endleistung (Pend,du) wird im 12. Zyklus in Stufe 12, (siehe Tabelle oben) bestimmt. Auch hier ist der Betriebspunkt 4000/min Volllast. Pend,du [kW] errechnet sich aus dem gemessenen Drehmoment.

Der Leistungsverlust im DU wird wie folgt berechnet

$$\text{Powerloss,du}\,[\%] = \left(1 - \frac{\text{Pend,du}}{\text{P0,du}}\right) * 100$$

Clean up

Die Anfangsleistung P0,cu [kW] wird aus dem gemessenen Drehmoment bei 4000/min Volllast direkt nach Teststart und Warmlauf des Motors im CU berechnet. Die Vorgehensweise ist ebenfalls in der Issue 5 der Testprozedur beschrieben.

Die Endleistung (Pend,cu) wird im 12. Zyklus in Stufe 12, (siehe Tabelle Figur 1) bestimmt. Auch hier ist der Betriebspunkt 4000/min Volllast. Pend,cu [kW] errechnet sich aus dem gemessenen Drehmoment.

Der Leistungsverlust im CU-Test wird wie folgt berechnet (negative Zahl beim powerloss im cu-Test bedeutet Leistungszuwachs)

$$\text{Powerloss (DU,CU)}\,[\%] = \left(\frac{\text{Pend,du} - \text{pend,cu}}{\text{P0,du}}\right) * 100$$

Als Kraftstoff wurde ein handelsüblicher Dieselkraftstoff der Fa. Haltermann (RF-06-03) eingesetzt. Diesem

wurden zur künstlichen Anregung der Bildung von Ablagerungen an den Injektoren 1 Gew.-ppm Zink in Form einer Zink-Didodecanoat-Lösung zugesetzt.

### 3. IDID Test - Bestimmung der Additivwirkung gegen interne Injektorablagerungen

Die Bildung von Ablagerungen im Inneren des Injektors wurde anhand der Abweichungen der Abgastemperaturen der Zylinder am Zylinderausgang beim Kaltstart des DW10-Motors charakterisiert.

Zur Förderung der Bildung von Ablagerungen wurden dem Kraftstoff 1 mg/l Na Salz einer organischen Säure, 20 mg/l Dodecenylbernsteinsäure und 10 mg/l Wasser zugegeben.

Der Test wird als dirty-up-clean-up Test (DU-CU) durchgeführt.

DU-CU lehnt sich an die an die CEC Test Prozedur F-098-08 Issue 5 an.

Der DU - CU Test besteht aus zwei einzelnen Tests, die hintereinander gefahren werden. Der erste Test dient zur Ablagerungsbildung (DU), der zweite zum Entfernen der Ablagerungen (CU).

Nach dem DU Lauf wird nach einer mindestens achtstündigen Stillstands-Phase ein Kaltstart des Motors mit anschließendem 10-minütigen Leerlauf durchgeführt.

Danach wird mit dem CU Kraftstoff der CU gestartet, ohne die Injektoren auszubauen und zu reinigen. Nach dem CU Lauf über 8h wird nach einer mindestens achtstündigen Stillstands-Phase ein Kaltstart des Motors mit anschließendem 10-minütigen Leerlauf durchgeführt. Die Auswertung erfolgt durch den Vergleich der Temperaturverläufe für die einzelnen Zylinder nach Kaltstart des du und des CU-Laufs.

Der IDID-Test zeigt die interne Ablagerungsbildung im Injektor an. Als Kenngröße dient bei diesem Test die Abgastemperatur der einzelnen Zylinder. Bei einem Injektor System ohne IDID erhöhen sich die Abgastemperaturen der Zylinder gleichmäßig. Bei vorhandenem IDID erhöhen sich die Abgastemperaturen der einzelnen Zylinder nicht gleichmäßig und weichen voneinander ab.

Die Temperatursensoren befinden sich hinter dem Zylinderkopfaustritt im Abgaskrümmer. Signifikante Abweichung der einzelnen Zylindertemperaturen (z.B. > 20°C) zeigen das Vorliegen von internen Injektorablagerungen (IDID) an.

Die Tests (DU und CU) werden mit jeweils 8h Laufzeit durchgeführt. Der Einstündige Testzyklus aus der CEC F-098-08 wird dabei jeweils 8-mal durchfahren. Bei Abweichungen der einzelnen Zylindertemperaturen von größer 45°C zum Mittelwert aller 4 Zylinder wird der Test vorzeitig abgebrochen.

## B. Herstellungsbeispiele:

### Herstellungsbeispiel 1: Herstellung von Polyisobutenepoxid

Zu einer Lösung von Polyisobuten 1000 (300 g) in n-Heptan (400 ml) werden bei Raumtemperatur Ameisensäure (96,6 g) und Methyltrioctylammoniumchlorid (1,33 g) gegeben. Das Reaktionsgemisch wird auf 75°C erhitzt und Wasserstoffperoxid-Lösung (30%, 88,4 g) wird langsam zugetropft, wobei die Temperatur bei 75°C gehalten wird. Das Reaktionsgemisch wird für 10 h bei 75°C gerührt, auf Raumtemperatur abgekühlt und die Phasen werden getrennt. Die organische Phase wird sukzessive mit wässriger $NaHSO_3$-Lösung gesättigter $NaHCO_3$-Lösung und Wasser gewaschen. Die organische Phase wird über $Na_2SO_4$ getrocknet und das Lösungsmittel wird mit Hilfe eines Rotationsverdampfers im Vakuum entfernt. Man erhält Polyisobuten-Epoxid (285 g).

### Herstellungsbeispiel 2: Polyisobutenepoxid - quaterniert mit N-Methylpyrrolidin/Essigsäure

(Referenzbeispiel)

Polyisobuten-Epoxid aus Herstellungsbeispiel 1 (30 g) wird mit Methanol (36,1 g) versetzt und N-Methylpyrrolidin (4,5 g) und Essigsäure (1,6 g) werden zugegeben. Das Reaktionsgemisch wird in einen Autoklaven überführt, mit Stickstoff inertisiert und unter Eigendruck für 20 h bei 140°C gerührt. Der Reaktorinhalt wird abgelassen und flüchtige Bestandteile werden mit Hilfe eines Rotationsverdampfers im Vakuum entfernt. Eine MALDI-MS-Analyse des so erhaltenen Reaktionsproduktes bestätigt die Bildung der quaternären Ammoniumverbindung.

### Herstellungsbeispiel 3: 1-Dodecenoxid - quaterniert mit N-Methylpyrrolidin/Essigsäure

Eine Lösung von 1-Dodecenoxid (73,7 g) und N-Methylpyrrolidin (97,2 g) in Isopropanol (193,7 g) wird bei 60° innerhalb von 10 Minuten mit Essigsäure (22,8 g) versetzt, und das so erhaltene Reaktionsgemisch wird für 14 h bei 60°C gerührt. Flüchtige Bestandteile werden mit Hilfe eines Rotationsverdampfers im Vakuum entfernt. Eine $^1$H-NMR-Analyse ($CECl_3$) des so erhaltenen Reaktionsproduktes (124 g) bestätigt die Bildung der quaternären Ammoniumverbindung ($\delta$

($NCH_3$) = 3,30 ppm).

**Herstellungsbeispiel 4: Gemisch aus 1-Dodecenoxid, 1-Tetradecenoxid und 1-Hexadecenoxid - quaterniert mit N-Methylpyrrolidin/Essigsäure**

Eine Lösung von 1-Dodecenoxid (18,4 g), 1-Tetradecenoxid (21,2 g), 1-Hexadecenoxid (24,0 g) und N-Methylpyrrolidin (51,1 g) in Isopropanol (133 g) wird bei 60° innerhalb von 10 Minuten mit Essigsäure (18,0 g) versetzt, und das erhaltene Reaktionsgemisch wird für 15 h bei 60°C gerührt. Flüchtige Bestandteile werden mit Hilfe eines Rotationsverdampfers im Vakuum entfernt. Eine $^1$H-NMR-Analyse (CDCb) des so erhaltenen Reaktionsproduktes (102 g) bestätigt die Bildung der quaternären Ammoniumverbindung ($\delta$ ($NCH_3$) = 3,30 ppm).

**Herstellungsbeispiel 5: 1-Dodecenoxid - quaterniert mit $NMe_3$/Tallölfettsäure**

In einem Autoklaven wird eine Lösung von 1-Dodecenoxid (19,4 g) in Isopropanol (59,4 g) bei Raumtemperatur mit Trimethylamin (11,8 g) versetzt. Das Gemisch wird unter Eigendruck auf 60°C erhitzt, Tallölfettsäure (28,2 g) wird langsam zudosiert und mit Isopropanol (10 ml) nachgespült. Das Reaktionsgemisch wird für 12 h bei 60°C nachgerührt. Der Reaktorinhalt wird abgekühlt und leicht flüchtige Bestandteile werden mit einem Stickstoffstrom ausgetrieben. Das Lösungsmittel wird anschließend mit Hilfe eines Rotationsverdampfers im Vakuum entfernt. Eine $^1$H-NMR-Analyse ($CECl_3$) des so erhaltenen Reaktionsproduktes (53,4 g) bestätigt die Bildung der quaternären Ammoniumverbindung ($\delta$ $N(CH_3)_3$) = 3,40 ppm).

**Herstellungsbeispiel 6: 1-Dodecenoxid - quaterniert mit N,N-Dimethylethanolamin/Tallölfettsäure**

Eine Lösung von 1-Dodecenoxid (73,6 g) und N,N-Dimethylethanolamin (34,2 g) in Isopropanol (214,8 g) wird bei 60°C innerhalb von 20 Minuten mit Tallölfettsäure (107 g) versetzt, und das so erhaltene Reaktionsgemisch wird für 22 h bei 60°C gerührt. Flüchtige Bestandteile werden mit Hilfe eines Rotationsverdampfers im Vakuum entfernt. Eine $^1$H-NMR-Analyse ($CDCl_3$) des so erhaltenen Reaktionsproduktes (212 g) bestätigt die Bildung der quaternären Ammoniumverbindung ($N(CH_3)_a$) = 3,33 ppm, $N(CH_3)_b$) = 3,34 ppm).

**Herstellungsbeispiel 7: 1-Dodecenoxid - quaterniert mit $NMe_3$/Salicylsäure**

In einem Autoklaven wird eine Lösung von 1-Dodecenoxid (23,9 g) in Isopropanol (57,1 g) bei Raumtemperatur mit Trimethylamin (15,3 g) versetzt. Das Gemisch wird unter Eigendruck auf 60°C erhitzt, eine Lösung von Salicylsäure (17,9 g) in Isopropanol (40 g) wird langsam zudosiert und mit Isopropanol (10 ml) nachgespült. Das Reaktionsgemisch wird für 12 h bei 60°C nachgerührt. Der Reaktorinhalt wird abgekühlt und leicht flüchtige Bestandteile werden mit einem Stickstoffstrom ausgetrieben. Das Lösungsmittel wird anschließend mit Hilfe eines Rotationsverdampfers im Vakuum entfernt. Eine $^1$H-NMR-Analyse ($CDCl_3$) des so erhaltenen Reaktionsproduktes (47,5 g) bestätigt die Bildung der quaternären Ammoniumverbindung ($N(CH_3)_3$) = 3,26 ppm).

**Herstellungsbeispiel 8: 1-Dodecenoxid - quaterniert mit N,N-Dimethylethanolamin/Salicylsäure**

Eine Lösung von 1-Dodecenoxid (73,6 g) und N,N-Dimethylethanolamin (34,2 g) in Isopropanol (160,3 g) wird bei 60°C innerhalb von 20 Minuten mit Salicylsäure (52,4 g) versetzt, und das so erhaltene Reaktionsgemisch wird für 23 h bei 60°C gerührt. Flüchtige Bestandteile werden mit Hilfe eines Rotationsverdampfers im Vakuum entfernt. Eine $^1$H-NMR-Analyse ($CDCl_3$) des so erhaltenen Reaktionsproduktes (154 g) bestätigt die Bildung der quaternären Ammoniumverbindung ($N(CH_3)_a$) = 3,21 ppm, $N(CH_3)_b$) = 3,22 ppm).

**C. Anwendungsbeispiele:**

In den folgenden Anwendungsbeispielen werden die Additive entweder als Reinsubstanz (so wie in obigen Herstellungsbeispielen synthetisiert) oder in Form eines Additiv-Paketes eingesetzt.

**Anwendungsbeispiel 1: Bestimmung der Additivwirkung auf die Bildung von Ablagerungen in Dieselmotor-Einspritzdüsen**

a) XUD9 Tests

Die Ergebnisse sind in Tabelle 1 zusammengefasst.

Tabelle 1: Ergebnisse der XUD9 Tests

| Bsp. | Bezeichnung | Kraftstoff | Dosierung ppm active | Flow restriction 0,1 mm Nadelhub [%] |
|---|---|---|---|---|
| #1 | gemäß Herstellungsbeispiel 3 | EN590-B7-1 | 40 | 14.3 |
| #2 | Grundwert | EN590-B7-1 | 0 | 72.5 |
| #3 | gemäß Herstellungsbeispiel 3 | RF-06-03 | 60 | 52.9 |
| #4 | Grundwert | RF-06-03 | 0 | 77.2 |
| #5 | gemäß Herstellungsbeispiel 4 | EN590-B7-1 | 40 | 7 |
| #6 | gemäß Herstellungsbeispiel 5 | EN590-B7-2 | 40 | 1.5 |
| #7 | Grundwert | EN590-B7-2 | 0 | 73.2 |
| #8 | gemäß Herstellungsbeispiel 2 | RF-06-03 | 40 | 0 |
| #9 | Grundwert | RF-06-03 | 0 | 68 |
| EN590-B7-1 und -2 sind kommerzielle Dieselkraftstoffe gemäß EN590 mit höchstens 7% Biodieselanteil gemäß DIN EN 14214. | | | | |

b) DW10 Dirty-up-Clean-up Test.

Tabelle 2: Ergebnisse der DW10 DU CUTests

| Bsp. | Bezeichnung | Kraftstoff | Dosierung ppm active | % Powerloss-DU, siehe Beschreibung | %Powerloss-DU-CU, siehe Beschreibung |
|---|---|---|---|---|---|
| #1 | gemäß Herstellungsbeispiel 4 | RF-06-03 | 105 | 4.1 | -6.1 |
| **step** | **duration (minutes)** | **engine speed (rpm) +/-20** | **load (%)** | **torque (Nm) +/-5** | **boost air after IC +/-3** |
| 1 | 2’ | 1750 | (20) | 62 | 45 |
| 2 | 7’ | 3000 | (60) | 173 | 50 |
| 3 | 2’ | 1750 | (20) | 62 | 45 |
| 4 | 7’ | 3500 | (80) | 212 | 50 |
| 5 | 2’ | 1750 | (20) | 62 | 45 |
| 6 | 10’ | 4000 | 100 | * | 50 |
| 7 | 2’ | 1250 | (10) | 25 | 43** |
| 8 | 7’ | 3000 | 100 | * | 50 |
| 9 | 2’ | 1250 | (10) | 25 | 43** |
| 10 | 10’ | 2000 | 100 | * | 50 |
| 11 | 2’ | 1250 | (10) | 25 | 43** |
| 12 | 7’ | 4000 | 100 | * | 50 |
| | ∑= 1 hour | | | | |
| * for expected ränge see appendix 06.5<br>** target only | | | | | |

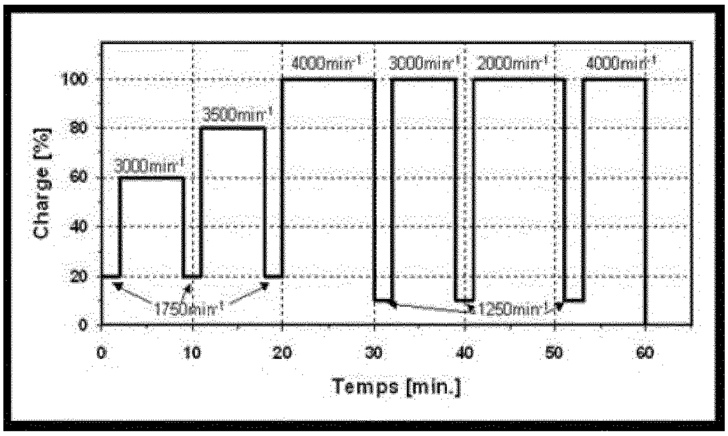


Fig. 1

## Patentansprüche

1. Verwendung eines eine quaternisierte Stickstoffverbindung umfassenden Reaktionsprodukts wobei das Reaktionsprodukt erhältlich ist durch Umsetzung wenigstens eines Hydrocarbylepoxids der allgemeinen Formel I

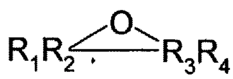

**(I)**

worin

wenigstens einer der Reste $R_1$ und $R_2$ für einen geradkettigen oder verzweigten, gesättigten oder ungesättigten langkettigen Hydrocarbylrest mit 7 bis 50 Kohlenstoffatomen steht und der andere der beiden Reste gegebenenfalls für H oder einen kurzkettigen Hydrocarbylrest, ausgewählt unter geradkettigem oder verzweigtem $C_1$-$C_7$-Alkyl oder $C_2$-$C_7$-Alkenyl, gegebenenfalls unterbrochen durch eine oder mehrere Heteroatomgruppen, oder gegebenenfalls ein- oder mehrfach substituiert, steht; und
die Reste $R_3$ und $R_4$ gleich oder verschieden sind und für H oder einen wie oben definierten kurzkettigen Hydrocarbylrest stehen;
mit wenigstens einem tertiären Amin der allgemeinen Formel II

$R_aR_bR_cN$ **(II)**

worin

$R_a$, $R_b$ und $R_c$ unabhängig voneinander für einen geradkettigen oder verzweigten, gesättigten oder ungesättigten, gegebenenfalls substituierten Hydrocarbylrest stehen, oder zwei der Reste $R_a$, $R_b$ und $R_c$, zusammen mit dem Stickstoffatom, an das sie gebunden sind, einen gegebenenfalls substituierten 5- bis 7-gliedrigen, gesättigten oder ungesättigten nicht-aromatischen oder aromatischen heterocyclischen Ring, der gegebenenfalls wenigstens ein weiteres Ringheteroatom, wie O, S oder N, tragen kann, bilden; wobei der Hydrocarbylrest im Amin der Formel II ein kurzkettiger Hydrocarbylrest ist und für geradkettiges oder verzweigtes $C_1$-$C_7$-Alkyl oder $C_2$-$C_7$-Alkenyl, gegebenenfalls unterbrochen durch eine oder mehrere Heteroatomgruppen, oder gegebenenfalls ein- oder mehrfach substituiert, steht;

sowie in Gegenwart wenigstens einer Säure der Formel III

$H^+A^-$ (III)

worin
A- für das Anion wenigstens einer ein- oder mehrwertigen, anorganischen oder organischen, natürlichen oder synthetischen Säure steht;
wobei die optionalen Substituenten der Reste ausgewählt sind unter -OH;
als Additiv zur Verringerung des Kraftstoffverbrauches von direkteinspritzenden Dieselmotoren, insbesondere von Dieselmotoren mit Common-Rail-Einspritzsystemen, und/oder zur Minimierung des Leistungsverlustes (powerloss) in direkteinspritzenden Dieselmotoren, insbesondere in Dieselmotoren mit Common-Rail-Einspritzsystemen.

2. Verwendung nach Anspruch 1, einer aus dem Reaktionsprodukt durch Aufreinigung erhaltenen, eine quaternisierte Stickstoffverbindung enthaltenden Teilfraktion, wobei diese wenigstens eine Verbindung der allgemeinen Formel IV

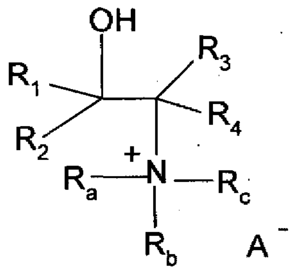

(IV)

umfasst, worin
die Reste $R_1$, $R_2$, $R_3$, $R_4$, $R_a$, $R_b$, und $R_c$ die oben angegebenen Bedeutungen besitzen und A für das Anion der eingesetzten Monocarbonsäure steht.

3. Verwendung nach einem der vorhergehenden Ansprüche, wobei das Reaktionsprodukt wenigstens eine Verbindung der allgemeinen Formel IV

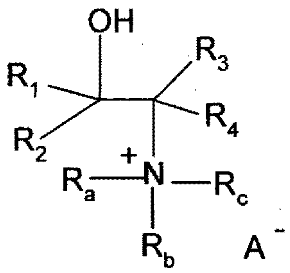

(IV)

umfasst, worin
die Reste $R_1$, $R_2$, $R_3$, $R_4$, $R_a$. $R_b$, und $R_c$ die oben angegebenen Bedeutungen besitzen und A für das Anion der eingesetzten Monocarbonsäure steht.

4. Verwendung nach einem der Ansprüche 1 bis 3, wobei in den Verbindungen der allgemeinen Formel I der langkettige Hydrocarbylrest für einen geradkettigen oder verzweigten, aliphatischen Kohlenwasserstoffrest mit 8 bis 40, insbesondere 10 bis 20 oder 12 bis 16 zusammenhängenden C-Atomen steht.

5. Verwendung eines eine quaternisierte Stickstoffverbindung umfassenden Reaktionsprodukts oder einer aus dem Reaktionsprodukt durch Aufreinigung erhaltenen, eine quaternisierte Stickstoffverbindung enthaltenden Teilfraktion davon, gemäß der Definition in einem der Ansprüche 1 bis 4 als Ottokraftstoffadditiv zur Verringerung von Ablagerungen im Einlasssystem eines Ottomotors, wie insbesondere DISI und PFI (Port Fuel Injector)-Motoren.

6. Verwendung eines eine quaternisierte Stickstoffverbindung umfassenden Reaktionsprodukts oder einer aus dem Reaktionsprodukt durch Aufreinigung erhaltenen, eine quaternisierte Stickstoffverbindung enthaltenden Teilfraktion davon, gemäß der Definition in einem der Ansprüche 1 bis 4 als Dieselkraftstoffadditiv zur Verringerung und/oder

Vermeidung von Ablagerungen in den Einspritzsystemen, wie insbesondere der Internal Diesel Injector Deposits (IDID) und / oder von Ventilkleben in direkteinspritzenden Dieselmotoren, insbesondere in Common-Rail-Einspritzsystemen.

## Claims

1. The use of a reaction product comprising a quaternized nitrogen compound, said reaction product being obtainable by

reacting at least one hydrocarbyl epoxide of the general formula I

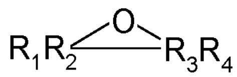

(I)

in which
at least one of the $R_1$ and $R_2$ radicals is a straight-chain or branched, saturated or unsaturated, long-chain hydrocarbyl radical having 7 to 50 carbon atoms and the other of the two radicals is optionally H or a short-chain hydrocarbyl radical selected from straight-chain or branched $C_1$-$C_7$-alkyl or $C_2$-$C_7$-alkenyl, optionally interrupted by one or more heteroatom groups, or optionally mono- or polysubstituted; and
the $R_3$ and $R_4$ radicals are the same or different and are each H or a short-chain hydrocarbyl radical as defined above;
with at least one tertiary amine of the general formula II

$R_aR_bR_cN$ (II)

in which
$R_a$, $R_b$ and $R_c$ are each independently a straight-chain or branched, saturated or unsaturated, optionally substituted hydrocarbyl radical, or two of the $R_a$, $R_b$ and $R_c$ radicals, together with the nitrogen atom to which they are bonded, form an optionally substituted 5- to 7-membered, saturated or unsaturated, nonaromatic or aromatic heterocyclic ring which may optionally bear at least one further ring heteroatom such as O, S or N; where the hydrocarbyl radical in the amine of the formula II is a short-chain hydrocarbyl radical and is straight-chain or branched $C_1$-$C_7$-alkyl or $C_2$-$C_7$-alkenyl, optionally interrupted by one or more heteroatom groups, or optionally mono- or polysubstituted;
and in the presence of at least one acid of the formula III

$H^+A^-$ (III)

in which
$A^-$ is the anion of at least one mono- or polybasic, inorganic or organic, natural or synthetic acid;
where the optional substituents of the radicals are selected from -OH;
as an additive for reducing the fuel consumption of direct injection diesel engines, especially of diesel engines with common rail injection systems, and/or for minimizing power loss in direct injection diesel engines, especially in diesel engines with common rail injection systems.

2. The use according to claim 1 of a fraction obtained from the reaction product by purification and comprising a quaternized nitrogen compound, wherein said fraction comprises at least one compound of the general formula IV

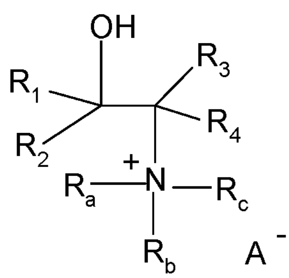

(IV)

in which

the $R_1$, $R_2$, $R_3$, $R_4$, $R_a$, $R_b$, and $R_c$ radicals are each as defined above and A is the anion of the monocarboxylic acid used.

**3.** The use according to either of the preceding claims, wherein the reaction product comprises at least one compound of the general formula IV

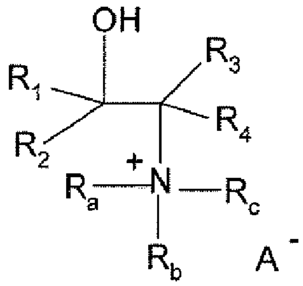

(IV)

in which
the $R_1$, $R_2$, $R_3$, $R_4$, $R_a$, $R_b$, and $R_c$ radicals are each as defined above and A is the anion of the monocarboxylic acid used.

**4.** The use according to any of claims 1 to 3, wherein the long-chain hydrocarbyl radical in the compounds of the general formula I is a straight-chain or branched aliphatic hydrocarbon radical having 8 to 40, especially 10 to 20 or 12 to 16 connected carbon atoms.

**5.** The use of a reaction product comprising a quaternized nitrogen compound, or of a fraction thereof obtained from the reaction product by purification and comprising a quaternized nitrogen compound, as defined in any of claims 1 to 4, as a gasoline fuel additive for reducing deposits in the intake system of a gasoline engine, such as, more particularly, DISI and PFI (port fuel injector) engines.

**6.** The use of a reaction product comprising a quaternized nitrogen compound, or of a fraction thereof obtained from the reaction product by purification and comprising a quaternized nitrogen compound, as defined in any of claims 1 to 4, as a diesel fuel additive for reducing and/or preventing deposits in the intake systems, such as especially the internal diesel injector deposits (IDIDs), and/or valve sticking in direct injection diesel engines, especially in common rail injection systems.

**Revendications**

**1.** Utilisation d'un produit de réaction comprenant un composé d'azote quaternisé, le produit de réaction pouvant être obtenu par

la mise en réaction d'au moins un époxyde d'hydrocarbyle de formule générale I

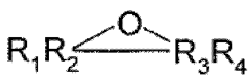

(I)

dans laquelle

au moins un des radicaux $R_1$ et $R_2$ représente un radical hydrocarbyle à chaîne longue linéaire ou ramifié, saturé ou insaturé, de 7 à 50 atomes de carbone, et l'autre des deux radicaux représente éventuellement H ou un radical hydrocarbyle à chaîne courte, choisi parmi alkyle en $C_1$-$C_7$ ou alcényle en $C_2$-$C_7$ linéaire ou ramifié, éventuellement interrompu par un ou plusieurs groupes d'hétéroatomes, ou éventuellement substitué une ou plusieurs fois ; et
les radicaux $R_3$ et $R_4$ sont identiques ou différents, et représentent H ou un radical hydrocarbyle à chaîne courte tel que défini ci-dessus ;

avec au moins une amine tertiaire de la formule générale II

$R_aR_bR_cN$ (II)

dans laquelle

$R_a$, $R_b$ et $R_c$ représentent indépendamment les uns des autres un radical hydrocarbyle linéaire ou ramifié, saturé ou insaturé, éventuellement substitué, ou deux des radicaux $R_a$, $R_b$ et $R_c$ forment ensemble avec l'atome d'azote auquel ils sont reliés un cycle hétérocyclique de 5 à 7 chaînons, saturé ou insaturé, non aromatique ou aromatique, éventuellement substitué, qui peut éventuellement porter au moins un hétéroatome de cycle supplémentaire, tel qu'O, S ou N ; le radical hydrocarbyle dans l'amine de formule II étant un radical hydrocarbyle à chaîne courte et représentant alkyle en $C_1$-$C_7$ ou alcényle en $C_2$-$C_7$ linéaire ou ramifié, éventuellement interrompu par un ou plusieurs groupes hétéroatomes, ou éventuellement substitué une ou plusieurs fois ;

ainsi qu'en présence d'au moins un acide de formule III

$H^+A^-$ (III)

dans laquelle

$A^-$ représente l'anion d'au moins un acide naturel ou synthétique, inorganique ou organique, mono- ou polyvalent ;

les substituants éventuels des radicaux étant choisis parmi -OH ;

en tant qu'additif pour réduire la consommation de carburant de moteurs diesel à injection directe, notamment de moteurs diesel à systèmes d'injection à rampe commune, et/ou pour minimiser la perte de puissance (power loss) dans des moteurs diesel à injection directe, notamment des moteurs diesel à systèmes d'injection à rampe commune.

2. Utilisation selon la revendication 1, d'une fraction partielle contenant un composé d'azote quaternisé obtenue à partir du produit de réaction par purification, celle-ci comprenant au moins un composé de formule générale IV

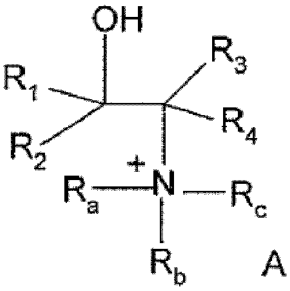

(IV)

dans laquelle

les radicaux $R_1$, $R_2$, $R_3$, $R_4$, $R_a$, $R_b$ et $R_c$ ont les significations indiquées ci-dessus et A représente l'anion de l'acide monocarboxylique utilisé.

3. Utilisation selon l'une quelconque des revendications précédentes, dans laquelle le produit de réaction comprend au moins un composé de formule générale IV

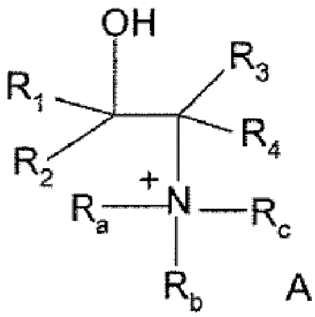

(IV)

dans laquelle

les radicaux $R_1$, $R_2$, $R_3$, $R_4$, $R_a$, $R_b$ et $R_c$ ont les significations indiquées ci-dessus et A représente l'anion de l'acide monocarboxylique utilisé.

**4.** Utilisation selon l'une quelconque des revendications 1 à 3, dans laquelle, dans les composés de la formule générale I, le radical hydrocarbyle à chaîne longue représente un radical hydrocarboné aliphatique linéaire ou ramifié comportant 8 à 40, notamment 10 à 20 ou 12 à 16 atomes de C connexes.

**5.** Utilisation d'un produit de réaction comprenant un composé d'azote quaternisé ou d'une fraction partielle de celui-ci contenant un composé d'azote quaternisé obtenu à partir du produit de réaction par purification, selon la définition dans l'une quelconque des revendications 1 à 4, en tant qu'additif pour carburant automobile pour réduire les dépôts dans le système d'entrée d'un moteur automobile, tel que notamment les moteurs DISI et PFI (Port Fuel Injector).

**6.** Utilisation d'un produit de réaction comprenant un composé d'azote quaternisé ou d'une fraction partielle de celui-ci contenant un composé d'azote quaternisé obtenu à partir du produit de réaction par purification, selon la définition dans l'une quelconque des revendications 1 à 4, en tant qu'additif pour carburant diesel pour réduire et/ou éviter les dépôts dans les systèmes d'injection, tels que notamment les dépôts internes d'injecteurs diesel (IDID) et/ou les adhérences au niveau de la soupape dans les moteurs diesel à injection directe, notamment dans les systèmes d'injection à rampe commune.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 4248719 A
- US 4171959 A
- EP 2033945 A
- WO 2006135881 A
- WO 2008060888 A
- WO 2007025700 A
- EP 1422246 A
- EP 244616 A
- WO 9424231 A
- WO 9703946 A
- DE 19620262 A
- WO 9603367 A
- WO 9603479 A
- EP 476485 A
- EP 307815 A
- WO 8701126 A
- EP 639632 A
- EP 310875 A
- EP 356725 A
- EP 700985 A
- US 4877416 A
- DE 3838918 A
- EP 831141 A
- DE 3826608 A
- DE 4142241 A
- DE 4309074 A
- EP 452328 A
- EP 548617 A
- DE 10102913 A
- WO 9929748 A
- WO 2005054314 A
- WO 2004035715 A
- EP 061895 A
- US 4491455 A
- WO 9318115 A
- EP 261957 A
- WO 0044857 A
- WO 98004656 A
- US 6743266 B2
- WO 0047698 A


### In der Beschreibung aufgeführte Nicht-Patentliteratur


- Epoxides. **SIENEL, G.** ; **RIETH, R.** ; **ROWBOTTOM, K. T.** Ullmann's Encyclopedia of Industrial Chemistry. 2000
- Polybutenes. **KOCH, H.** ; **MAWER, R. L.** ; **IMMEL, W.** Ullmann's Encyclopedia of Industrial Chemistry. 2011
- **JAMES L. WHITE** ; **DONGMAN CHOI**. Polyolefins: Processing, Structure Development, and Properties. Hanser, 2004
- **N. A. PLATE** ; **V. P. SHIBAEV**. Comb-Like Polymers. Structure and Properties. *J. Poly. Sci. Macromolecular Revs.*, 1974, vol. 8, 117-253
- Amines, aliphatic. Ullmanns Encyclopedia of Industrial Chemistry
- Ullmann' s Encyclopedia of Industrial Chemistry, vol. A12, 617 ff
- *CHEMICAL ABSTRACTS*, 64-18-6
- *CHEMICAL ABSTRACTS*, 142-82-5
- *CHEMICAL ABSTRACTS*, 67-56-1
- *CHEMICAL ABSTRACTS*, 64-19-7
- *CHEMICAL ABSTRACTS*, 120-94-5
- *CHEMICAL ABSTRACTS*, 2855-19-8
- *CHEMICAL ABSTRACTS*, 3234-28-4
- *CHEMICAL ABSTRACTS*, 7320-37-8
- *CHEMICAL ABSTRACTS*, 67-63-0
- *CHEMICAL ABSTRACTS*, 75-50-3
- *CHEMICAL ABSTRACTS*, 108-01-0
- *CHEMICAL ABSTRACTS*, 69-72-7